# EUROPEAN PATENT APPLICATION

(11) **EP 1 391 518 A1**
(43) Date of publication of application: **25.02.2004**
(21) Application number: 02769430.6
(22) Date of filing: 16.05.2002
(51) Int. Cl.: C12P 21/02, C12N 15/09, C07K 19/00, C12N 1/21

(54) **PROCESS FOR PRODUCING PEPTIDE**

(30) Priority: 17.05.2001 JP 2001147341
(71) Applicant: Takeda Chemical Industries, Ltd., Osaka-shi, Osaka 541-0045 (JP)
(72) Inventor: NISHIMURA, Osamu, Kawanishi-shi, Hyogo 666-0112 (JP); SUENAGA, Masato, Tokuyama-shi, Yamaguchi 745-0844 (JP); ITO, Takashi, Tsukuba-shi, Ibaraki 305-0821 (JP); KITADA, Chieko, Sakai-shi, Osaka 590-0073 (JP)
(74) Representative: Pohlmann, Eckart, Dipl.-Phys.
(86) International application number: PCT/JP2002/004735
(87) International publication number: WO 2002/092829

(57) **Abstract**

It is an objective of the present invention to provide a novel gene recombination-based method for producing desired peptides that allows effective, large scale production of the desired peptides.

According to the method of the present invention, excision of peptides of interest is achieved by the use of the right-handed scissors (*i.e.*, S-cyanylation reaction) along with the left-handed scissors (*i.e.*, treatment with cyanogen bromide, enterokinase, factor Xa, or the like). The method utilizes the excision technique in conjunction with the tandem repeat technique and thereby provides a useful gene recombination-based technique for peptide synthesis that is particularly effective in the large-scale synthesis of low-molecular weight peptides.

[Sequence Listing]

<110> Takeda Chemical Industries, Ltd.

<120> A Method for Producing A Peptide

<130> P02-0061PCT

<150> JP 2001-147341
<151> 2001-05-17

<160> 75

<210> 1
<211> 54
<212> PRT
<213> Human

<220>
<223> the C-terminus of the polypeptide is amide (-CONH2) form

<400> 1
Gly Thr Ser Leu Ser Pro Pro Pro Glu Ser Ser Gly Ser Arg Gln Gln
1               5                   10                  15
Pro Gly Leu Ser Ala Pro His Ser Arg Gln Ile Pro Ala Pro Gln Gly

20                   25                   30
Ala Val Leu Val Gln Arg Glu Lys Asp Leu Pro Asn Tyr Asn Trp Asn
        35                   40                   45
Ser Phe Gly Leu Arg Phe
    50

<210> 2
<211> 10
<212> PRT
<213> Human

<220>
<223> the C-terminus of the polypeptide is amide (-CONH2) form

<400> 2
Tyr Asn Trp Asn Ser Phe Gly Leu Arg Phe
                5                   10

<210> 3
<211> 162
<212> DNA
<213> Human

<400> 3
ggtacttctc tgtctccgcc gccggaatct tctggttctc gtcagcagcc gggtctgtct    60

gctccgcact ctcgtcagat cccggctccg cagggtgctg ttctggttca gcgtgaaaaa   120

gacctgccga actacaactg gaactctttc ggtctgcgtt tc                        162

<210> 4

<211> 30

<212> DNA

<213> Human

<400> 4

tataactgga acagctttgg tctgcgtttt                        30

<210> 5

<211> 144

<212> DNA

<213> Human

<400> 5

ggtagcgcga tgtataactg gaacagcttt ggtctgcgtt tttgtggctc ggcgatgtac  60

aattggaatt ccttcggcct gcgcttctgc ggctcggcga tgtataactg gaactccttt 120

ggcctgcgct tttgcggttc tgct                                        144

<210> 6

<211> 9

<212> PRT

<213> Human

<400> 6

Ser Phe Ala Asn Leu Pro Leu Arg Phe

1               5

<210> 7

<211> 12

<212> PRT

<213> Human

<400> 7

Met Pro His Ser Phe Ala Asn Leu Pro Leu Arg Phe

1               5                   10

<210> 8

<211> 20

<212> PRT

<213> Human

<400> 8

Met Ser Thr Pro Ala Val Asn Lys Met Pro His Ser Phe Ala Asn Leu

1               5                   10                  15

Pro Leu Arg Phe

            20

<210> 9 .

<211> 37

<212> PRT

<213> Human

<400> 9

Ser Leu Asn Phe Glu Glu Leu Lys Asp Trp Gly Pro Lys Asn Val Ile

1               5                   10                  15

Lys Met Ser Thr Pro Ala Val Asn Lys Met Pro His Ser Phe Ala Asn

            20                  25                  30

Leu Pro Leu Arg Phe

        35

<210> 10

<211> 8

<212> PRT

<213> Human

<400> 10

Val Pro Asn Leu Pro Gln Arg Phe

1               5

<210> 11

<211> 17

<212> PRT

<213> Human

<400> 11

Asn Met Glu Val Ser Leu Val Arg Arg Val Pro Asn Leu Pro Gln Arg

1               5                   10                  15

Phe

<210> 12

<211> 27

<212> DNA

<213> Human

<400> 12

agctttgcga atctgccgct gcgtttt          27

<210> 13

<211> 36

<212> DNA

<213> Human

<400> 13

atgccgcata gctttgcgaa tctgccgctg cgtttt       36

<210> 14

<211> 60

<212> DNA

<213> Human

<400> 14

atgagcaccc cggcggtgaa taaaatgccg catagctttg cgaatctgcc gctgcgtttt    60

<210> 15

<211> 111

<212> DNA

<213> Human

<400> 15

agcctgaact ttgaagaact gaaagattgg ggtccgaaaa atgtgattaa aatgagcacc    60

ccggcggtga ataaaatgcc gcatagcttt gcgaatctgc cgctgcgttt t    111

<210> 16

<211> 24

<212> DNA

<213> Human

<400> 16

gttcctaacc tgccccaaag gttt    24

<210> 17

<211> 51

<212> DNA

<213> Human

<400> 17

aatatggagg tgagcctcgt gagacgtgtt cctaacctgc cccaaaggtt t 51

<210> 18

<211> 36

<212> PRT

<213> Human

<400> 18

Leu Val Gln Pro Arg Gly Ser Arg Asn Gly Pro Gly Pro Trp Gln Gly

1               5                   10                  15

Gly Arg Arg Lys Phe Arg Arg Gln Arg Pro Arg Leu Ser His Lys Gly

            20                  25                  30

Pro Met Pro Phe

        35

<210> 19

<211> 108

<212> DNA

<213> Human

<400> 19

ctggtgcagc ccagagggtc aaggaatggg ccagggccct ggcagggagg tcggaggaaa 60

ttccgccgcc agcggccccg cctctcccat aagggaccca tgcctttc 108

<210> 20

<211> 31

<212> PRT

<213> Bovine

<400> 20

Ser Arg Ala His Gln His Ser Met Glu Ile Arg Thr Pro Asp Ile Asr

1 5 10 15

Pro Ala Trp Tyr Ala Gly Arg Gly Ile Arg Pro Val Gly Arg Phe

20 25 30

<210> 21

<211> 31

<212> PRT

<213> RAT

<400> 21

Ser Arg Ala His Gln His Ser Met Glu Thr Arg Thr Pro Asp Ile Asn

1               5                   10                  15

Pro Ala Trp Tyr Thr Gly Arg Gly Ile Arg Pro Val Gly Arg Phe

20                    25                    30

<210> 22

<211> 31

<212> PRT

<213> Human

<400> 22

Ser Arg Thr His Arg His Ser Met Glu Ile Arg Thr Pro Asp Ile Asn

1               5                   10                  15

Pro Ala Trp Tyr Ala Ser Arg Gly Ile Arg Pro Val Gly Arg Phe

            20                  25                  30

<210> 23

<211> 21

<212> PRT

<213> Bovine

<220>

<221>

<222>

<223>  10 th Xaa is Ala or Thr,  11 th Xaa is Gly or Ser and 21th Xaa is OH, Gly or

GlyArg.

<400> 23

Thr Pro Asp Ile Asn Pro Ala Trp Tyr Xaa Xaa Arg Gly Ile Arg Pro
1               5                   10                  15
Val Gly Arg Phe Xaa
            20

<210> 24
<211> 29
<212> PRT
<213> Bovine

<400> 24
Ser Arg Ala His Gln His Ser Met Glu Ile Arg Thr Pro Asp Ile Asn
1               5                   10                  15
Pro Ala Trp Tyr Ala Gly Arg Gly Ile Arg Pro Val Gly
            20                  25

<210> 25
<211> 19
<212> PRT
<213> Bovine

<400> 25
Thr Pro Asp Ile Asn Pro Ala Trp Tyr Ala Gly Arg Gly Ile Arg Pro
1               5                   10                  15
Val Gly Arg

19

<210> 26

<211> 31

<212> PRT

<213> Bovine

<400> 26

Ser Arg Ala His Gln His Ser Met Glu Ile Arg Thr Pro Asp Ile Asn

1               5                   10                  15

Pro Ala Trp Tyr Ala Gly Arg Gly Ile Arg Pro Val Gly Arg Phe

            20                  25                  30

<210> 27

<211> 32

<212> PRT

<213> Bovine

<400> 27

Ser Arg Ala His Gln His Ser Met Glu Ile Arg Thr Pro Asp Ile Asn

1               5                   10                  15

Pro Ala Trp Tyr Ala Gly Arg Gly Ile Arg Pro Val Gly Arg Phe Gly

            20                  25                  30

<210> 28

<211> 33

<212> PRT

<213> Bovine

<400> 28

Ser Arg Ala His Gln His Ser Met Glu Ile Arg Thr Pro Asp Ile Asn

1               5                   10                  15

Pro Ala Trp Tyr Ala Gly Arg Gly Ile Arg Pro Val Gly Arg Phe Gly

            20                  25                  30

Arg

<210> 29

<211> 20

<212> PRT

<213> Bovine

<400> 29

Thr Pro Asp Ile Asn Pro Ala Trp Tyr Ala Gly Arg Gly Ile Arg Pro

1               5                   10                  15

Val Gly Arg Phe

            20

<210> 30

<211> 21

<212> PRT

<213> Bovine

<400> 30

Thr Pro Asp Ile Asn Pro Ala Trp Tyr Ala Gly Arg Gly Ile Arg Pro

1               5                   10                  15

Val Gly Arg Phe Gly

            20

<210> 31

<211> 22

<212> PRT

<213> Rat

<400> 31

Thr Pro Asp Ile Asn Pro Ala Trp Tyr Ala Gly Arg Gly Ile Arg Pro

1               5                   10                  15

Val Gly Arg Phe Gly Arg

            20

<210> 32

<211> 31

<212> PRT

<213> Rat

<400> 32

Ser Arg Ala His Gln His Ser Met Glu Thr Arg Thr Pro Asp Ile Asn

1               5                   10                  15

Pro Ala Trp Tyr Thr Gly Arg Gly Ile Arg Pro Val Gly Arg Phe

            20                  25                  30

<210> 33

<211> 32

<212> PRT

<213> Rat

<400> 33

Ser Arg Ala His Gln His Ser Met Glu Thr Arg Thr Pro Asp Ile Asn

1               5                   10                  15

Pro Ala Trp Tyr Thr Gly Arg Gly Ile Arg Pro Val Gly Arg Phe Gly

            20                  25                  30

<210> 34

<211> 33

<212> PRT

<213> Rat

<400> 34

Ser Arg Ala His Gln His Ser Met Glu Thr Arg Thr Pro Asp Ile Asn

1               5                   10                  15

Pro Ala Trp Tyr Thr Gly Arg Gly Ile Arg Pro Val Gly Arg Phe Gly
            20                  25                  30
Arg

<210> 35
<211> 20
<212> PRT
<213> Rat

<400> 35
Thr Pro Asp Ile Asn Pro Ala Trp Tyr Thr Gly Arg Gly Ile Arg Pro
1               5                   10                  15
Val Gly Arg Phe
            20

<210> 36
<211> 21
<212> PRT
<213> Rat

<400> 36
Thr Pro Asp Ile Asn Pro Ala Trp Tyr Thr Gly Arg Gly Ile Arg Pro
1               5                   10                  15
Val Gly Arg Phe Gly
            20

<210> 37

<211> 22

<212> PRT

<213> Human

<400> 37

Thr Pro Asp Ile Asn Pro Ala Trp Tyr Thr Gly Arg Gly Ile Arg Pro

1               5                   10                  15

Val Gly Arg Phe Gly Arg

            20

<210> 38

<211> 31

<212> PRT

<213> Human

<400> 38

Ser Arg Thr His Arg His Ser Met Glu Ile Arg Thr Pro Asp Ile Asn

1               5                   10                  15

Pro Ala Trp Tyr Ala Ser Arg Gly Ile Arg Pro Val Gly Arg Phe

            20                  25                  30

<210> 39

<211> 32

<212> PRT

<213> Human

<400> 39

Ser Arg Thr His Arg His Ser Met Glu Ile Arg Thr Pro Asp Ile Asn

1               5                   10                  15

Pro Ala Trp Tyr Ala Ser Arg Gly Ile Arg Pro Val Gly Arg Phe Gly

            20                  25                  30

<210> 40

<211> 33

<212> PRT

<213> Human

<400> 40

Ser Arg Thr His Arg His Ser Met Glu Ile Arg Thr Pro Asp Ile Asn

1               5                   10                  15

Pro Ala Trp Tyr Ala Ser Arg Gly Ile Arg Pro Val Gly Arg Phe Gly

            20                  25                  30

Arg

<210> 41

<211> 20

<212> PRT

<213> Human

<400> 41

Thr Pro Asp Ile Asn Pro Ala Trp Tyr Ala Ser Arg Gly Ile Arg Pro

1               5                   10                  15

Val Gly Arg Phe

            20

<210> 42

<211> 21

<212> PRT

<213> Human

<400> 42

Thr Pro Asp Ile Asn Pro Ala Trp Tyr Ala Ser Arg Gly Ile Arg Pro

1               5                   10                  15

Val Gly Arg Phe Gly

            20

<210> 43

<211> 22

<212> PRT

<213> Human

<400> 43

Thr Pro Asp Ile Asn Pro Ala Trp Tyr Ala Ser Arg Gly Ile Arg Pro

1                    5                        10                       15

Val Gly Arg Phe Gly Arg

                20

<210> 44

<211> 23

<212> PRT

<213> Human

<400> 44

Trp Tyr Lys His Val Ala Ser Pro Arg Tyr His Thr Val Gly Arg Ala

1                    5                        10                       15

Ala Gly Leu Leu Met Gly Leu

                20

<210> 45

<211> 23

<212> PRT

<213> Porcine

<400> 45

Trp Tyr Lys His Thr Ala Ser Pro Arg Tyr His Thr Val Gly Arg Ala

1                    5                        10                       15

Ala Gly Leu Leu Met Gly Leu

                20

<210> 46

<211> 23

<212> PRT

<213> Rat/Mouse

<400> 46

Trp Tyr Lys His Val Ala Ser Pro Arg Tyr His Thr Val Gly Arg Ala

1               5                   10                  15

Ser Gly Leu Leu Met Gly Leu

            20

<210> 47

<211> 30

<212> PRT

<213> Human

<400> 47

Trp Tyr Lys His Val Ala Ser Pro Arg Tyr His Thr Val Gly Arg Ala

1               5                   10                  15

Ala Gly Leu Leu Met Gly Leu Arg Arg Ser Pro Tyr Leu Trp

            20                  25                  30

<210> 48

<211> 30

<212> PRT

<213> Porcine

<400> 48

Trp Tyr Lys His Thr Ala Ser Pro Arg Tyr His Thr Val Gly Arg Ala

1               5                   10                  15

Ala Gly Leu Leu Met Gly Leu Arg Arg Ser Pro Tyr Met Trp

            20                  25                  30

<210> 49

<211> 30

<212> PRT

<213> Rat

<400> 49

Trp Tyr Lys His Val Ala Ser Pro Arg Tyr His Thr Val Gly Arg Ala

1               5                   10                  15

Ser Gly Leu Leu Met Gly Leu Arg Arg Ser Pro Tyr Leu Trp

            20                  25                  30

<210> 50

<211> 30

<212> PRT

<213> Mouse

<400> 50

Trp Tyr Lys His Val Ala Ser Pro Arg Tyr His Thr Val Gly Arg Ala

1               5                   10                  15

Ser Gly Leu Leu Met Gly Leu Arg Arg Ser Pro Tyr Gln Trp

            20                  25                  30

<210> 51

<211> 69

<212> DNA

<213> Human

<400> 51

tggtacaagc acgtggcgag tccccgctac cacacggtgg gccgcgccgc tggcctgctc    60

atggggctg                                                            69

<210> 52

<211> 69

<212> DNA

<213> Porcine

<400> 52

tggtacaagc acacggcgag tccccgctac cacacggtgg gccgcgccgc gggcctgctc    60

atggggctg                                                            69

<210> 53

<211> 69

<212> DNA

<213> Rat/Mouse

<400> 53

tggtacaagc acgtggcgag ccctcgctat cacacagtgg gtcgtgcctc cgggctgctc    60

atggggctg                                                            69

<210> 54

<211> 90

<212> DNA

<213> Human

<400> 54

tggtacaagc acgtggcgag tccccgctac cacacggtgg gccgcgccgc tggcctgctc    60

atggggctgc gtcgctcacc ctatctgtgg                                     90

<210> 55

<211> 90

<212> DNA

<213> Porcine

<400> 55

tggtacaagc acacggcgag tccccgctac cacacggtgg gccgcgccgc gggcctgctc    60

atggggctgc gccgctcgcc ctacatgtgg                                     90

<210> 56

<211> 90

<212> DNA

<213> Rat

<400> 56

tggtacaagc acgtggcgag ccctcgctat cacacagtgg gtcgtgcctc cgggctgctc 60

atggggctgc gccgctcgcc ctacctgtgg 90

<210> 57

<211> 90

<212> DNA

<213> Mouse

<400> 57

tggtataagc acgtggcgag tccccgctat cacacagtgg gtcgtgcctc cgggctgctc 60

atggggctgc gccgctcgcc ctaccagtgg 90

<210> 58

<211> 5

<212> PRT

<213> Artificial Sequence

<220>

<223>

<400> 58

Asp Asp Asp Asp Lys

1               5

<210> 59

<211> 15

<212> DNA

<213> Artificial Sequence

<220>

<223>

<400> 59

gatgacgacg acaag                                                    15

<210> 60

<211> 4

<212> PRT

<213> Artificial Sequence

<220>

<223>

<400> 60

Ile Glu Gly Arg

1

<210> 61

<211> 12

<212> DNA

<213> Artificial Sequence

<220>

<223>

<400> 61

attgaaggcc gc                                    12

<210> 62

<211> 3

<212> PRT

<213> Artificial Sequence

<220>

<223>

<400> 62

Gly Pro Arg

1

<210> 63

<211> 9

<212> DNA

<213> Artificial Sequence

<220>

<223>

<400> 63

ggcccgcgc 9

<210> 64

<211> 288

<212> DNA

<213> Human

<400> 64

gatgacgatg acaaatggta taaacatgtg gcgagcccgc gttatcatac cgtgggccgc 60

gcggccggtc tgctgatggg cctgtgtcaa ttgggtggtg atgacgatga caaatggtat 120

aaacatgtgg cgagcccgcg ttatcatacc gtgggccgcg cggccggtct gctgatgggc 180

ctgtgtgagc tcggctctga cgacgatgat aaatggtaca aacacgttgc ctccccgcgc 240

taccacacgg ttggtcgtgc cgcgggcctg ctgatgggtc tgtgcggt 288

<210> 65

<211> 56

<212> DNA

<213> Artificial Sequence

<220>

<223> oligomer

<400> 65

tatggatgac gatgacaaat ggtataaaca tgtggcgagc ccgcgttatc ataccg 56

<210> 66

<211> 64

<212> DNA

<213> Artificial Sequence

<220>

<223> oligomer

<400> 66

gcgcggccca cggtatgata acgcgggctc gccacatgtt tataccattt gtcatcgtca 60

tcca 64

<210> 67

<211> 72

<212> DNA

<213> Artificial Sequence

<220>

<223> oligomer

<400> 67

tgggccgcgc ggccggtctg ctgatgggcc tgtgtcaatt gggtttgaac ttctctgtct 60

ccgccgccgg ag 72

<210> 68

<211> 66

<212> DNA

<213> Artificial Sequence

<220>

<223> oligomer

<400> 68

gatcctccgg cggcggagac agagaagttc aaacccaatt gacacaggcc catcagcaga 60

ccggcc 66

<210> 69

<211> 63

<212> DNA

<213> Artificial Sequence

<220>

<223> oligomer

<400> 69

aattgggtgg tgatgacgat gacaaatggt ataaacatgt ggcgagcccg cgttatcata 60

ccg 63

<210> 70

<211> 66

<212> DNA

<213> Artificial Sequence

<220>

<223> oligomer

<400> 70

cggcccacgg tatgataacg cgggctcgcc acatgtttat accatttgtc atcgtcatca 60

ccaccc 66

<210> 71

<211> 68

<212> DNA

<213> Artificial Sequence

<220>

<223> oligomer

<400> 71

tgggccgcgc ggccggtctg ctgatgggcc tgtgtgagct cggctctgac gacgatgata 60

aatggtac 68

<210> 72

<211> 68

<212> DNA

<213> Artificial Sequence

<220>

<223> oligomer

<400> 72

caacgtgttt gtaccattta tcatcgtcgt cagagccgag ctcacacagg cccatcagca 60

gaccggcc 68

<210> 73

<211> 73

<212> DNA

<213> Artificial Sequence

<220>

<223> oligomer

<400> 73

aaacacgttg cctccccgcg ctaccacacg gttggtcgtg ccgcgggcct gctgatgggt 60

ctgtgcggtt gag 73

<210> 74

<211> 67

<212> DNA

<213> Artificial Sequence

<220>

<223> oligomer

<400> 74

gatcctcaac cgcacagacc catcagcagg cccgcggcac gaccaaccgt gtggtagcgc 60

ggggagg 67

## Description

### TECHNICAL FIELD

The present invention relates to a method for producing a peptide of interest or a salt thereof. In this method, a precursor protein containing a plurality of peptides of interest that are linked in a repetitive fashion is stably expressed in microorganisms and the peptide bonds of the expressed precursor protein are subsequently cleaved to obtain the desired peptide or the salt thereof.

### BACKGROUND ART

In the synthesis of peptides, three different approaches are available: organic chemical approach, enzymatic approach, and gene recombination approach.

With regard to the gene recombination approach, peptide synthesis by the direct expression of peptides is extremely difficult since peptides are immediately subjected to digestion by proteases as they are expressed in the cells of microorganisms.

For this reason, the fusion protein technique is currently the most commonly used technique in the gene recombination-based synthesis of peptides. In this technique, a peptide is expressed as a fusion protein fused to a protective protein, such as protein A and beta-galactosidase, that can be readily separated by affinity chromatography and can thus allows rapid and efficient purification of the peptides. The desired peptide must then be excised from the fusion protein. Among known techniques used for this purpose are chemical methods, such as one involving the use of cyanogen bromide (which cleaves at the C-terminal side of Met), and enzymatic methods, by which proteins are cleaved immediately after the C-terminal ends of particular amino acid sequences such as enterokinase cleavage site (Asp-Asp-Asp-Asp-Lys) and factor Xa cleavage site (Ile-Glu-Gly-Arg).

A novel peptide synthesis technique is also reported in which an FGF mutein (CS23) to serve as a protective protein for a fusion protein is used in combination with S-cyanylation reaction, a specific chemical cleavage reaction (EP0887417). Not only does this technique exploit the strong affinity of CS23 to heparin to facilitate the purification of the fusion protein, but the S-cyanylation reaction, which specifically cleaves the protein at the N-terminal side of a cysteine residue, also allows the excision of the desired peptide. Furthermore, C-terminal amide, a form of peptide that a peptide must be converted into to exhibit its physiological activities, can be readily prepared by this method. In addition, the method is applicable to virtually any cysteine-free peptide.

Nonetheless, fusion protein techniques also have a drawback: large molecular weight differences between protective proteins and desired peptides make it difficult to fully exploit the ability of microbes (*e.g.*, E. coli) to synthesize proteins. In an attempt to cope with this problem, the tandem repeat technique has been developed. In this technique, genes of peptides of interest are linked in tandem within one molecule to be expressed as a stable precursor protein in the cells of microorganisms, and the peptides of interest are subsequently excised from the protein. Few successful examples of this technique have so far been reported, however. What makes the technique impractical is that although several means, including the cyanogen bromide treatment and the factor Xa technique, have previously been known for cleaving on the N-terminal side of each peptide to expose the N-terminal (left-handed scissors, so to speak), no effective means has been available for specifically cleaving on the C-terminal side of the peptide to expose the C-terminal (right-handed scissors, so to speak).

Thus, it is an objective of the present invention to provide a novel gene recombination-based method for producing desired peptides that allows effective, large scale production of the desired peptides.

### DISCLOSURE OF THE INVENTION

In the course of studies on the technique for specifically cleaving on the C-terminal side to expose the C-terminal, the present inventors have unexpectedly discovered that the above-described S-cyanylation reaction finds its application not only in the fusion protein technique, but also in the peptide synthesis utilizing the tandem repeat technique. The discovery led the present inventors to devise the present invention.

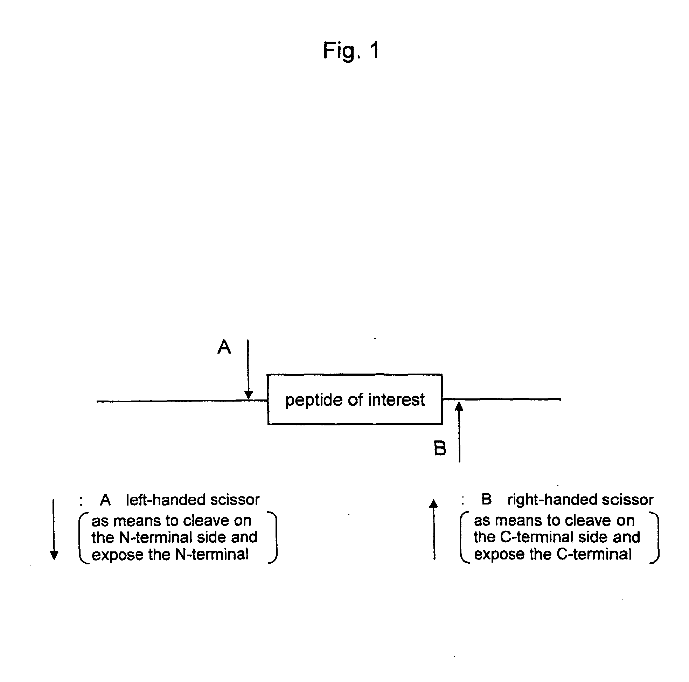

Specifically, the present inventors successfully obtained both the left-handed scissors and the right-handed scissors, the tools required to make the tandem repeat technique practical. As a result, effective, large-scale preparation of peptides, in particular those with small molecular weights, which have been considered difficult to synthesize by the conventional gene recombination approaches, has become possible for the first time (Fig. 1).

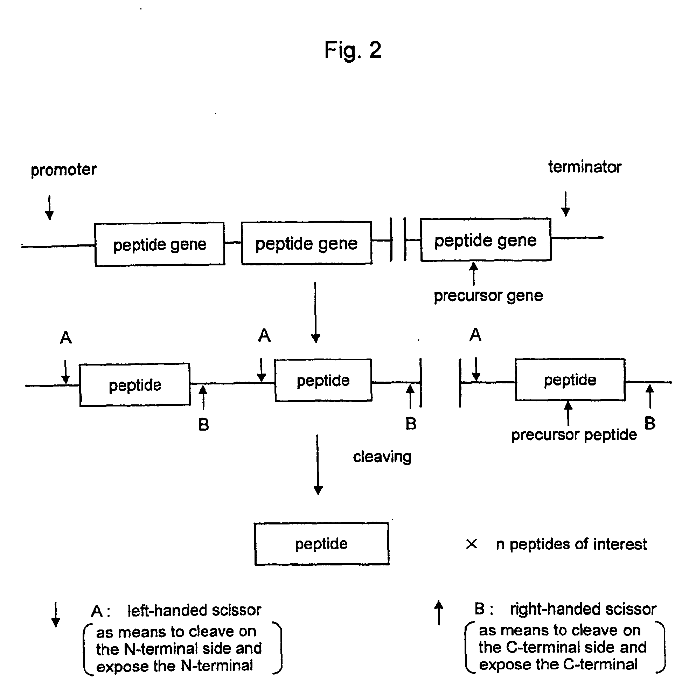

As described above, the combination of the two cutting means allows effective, large-scale preparation of small molecular weight peptides by the tandem repeat technique (Fig. 2).

According the present invention, there are provided:
(1) A process for producing a peptide of interest or a salt thereof, characterized in that:
   a precursor protein, containing repetitive links of the peptides of interest, each of which has an enzymatic or chemical cleavage site added to its N-terminal end and C-terminal end to repetitively link, is enzymatically or chemically cleaved. (Production Method A, hereinafter);
(2) The process according to (2) above, characterized in that:
   the precursor protein containing repetitive links of the peptides of interest, each of which has an enzymatic or chemical cleavage site added to its N-terminal end and a chemical cleavage site added to its C-terminal end to repetitively link, is enzymatically or chemically cleaved;
(3) A process for producing a peptide of interest or a salt thereof, characterized in that:
   a precursor protein containing repetitive links of the peptides of interest, each of which has a methionine residue or a protease cleavage sequence added to its N-terminal end and a cysteine residue or a cysteinyl peptide added to its C-terminal end to repetitively link (wherein the peptide moiety of the cysteinyl peptide is a peptide different from the peptide of interest and does not contain any methionine residue when a methionine residue is added to the N-terminal end of each peptide of interest), is cleaved by cyanogen bromide or a protease on the N-terminal side of each peptide and is subjected to a cleavage reaction to cleave on the N-terminal side of each of the C-terminal cysteine residues or the cysteinyl peptides;
(4) The process according to (1) to (3) above, wherein the precursor protein is a recombinant precursor protein;
(5) The process according to (3) above, wherein the cleavage reaction comprises S-cyanylation reaction, followed by ammonolysis or hydrolysis;
(6) The process according to (5) above, wherein the S-cyanylation reaction is carried out in the presence of 2-nitro-5-thiocyanobenzoic acid (NTCB), a 1-cyano-4-dimethylamino pyridium salt (DMAP-CN), or CN⁻ ion;
(7) The process according to (3) above, wherein the protease is enterokinase, factor Xa, or thrombin;
(8) The process according to (3) above, wherein the following conditions are met:
   (i) in cases where cyanogen bromide is used, a methionine residue is linked to the N-terminal end of each peptide of interest and any of the peptides of interest does not contain a methionine residue;
   (ii) in cases where the protease is enterokinase, an amino acid sequence Asp-Asp-Asp-Asp-Lys is linked to the N-terminal end of each peptide of interest and any of the peptides of interest does not contain the amino acid sequence Asp-Asp-Asp-Asp-Lys;
   (iii) in cases where the protease is factor Xa, an amino acid sequence Ile-Glu-Gly-Arg is linked to the N-terminal end of each peptide of interest and any of the peptides of interest does not contain the amino acid sequence Ile-Glu-Gly-Arg; and
   (iv) in cases where the protease is thrombin, an amino acid sequence Gly-Pro-Arg is linked to the N-terminal end of each peptide of interest and any of the peptides of interest does not contain the amino acid sequence Gly-Pro-Arg;
(9) The process according to (1) to (3) above, wherein the peptide of interest is peptide KiSS-1;
(10) The process according to (1) to (3) above, wherein the peptide of interest is a ligand for GPR8;
(11) A process for producing a GPR8 ligand or a salt thereof, characterized in that:
   a precursor protein containing three repetitive likings of the GPR8 ligands, each of which has an enterokinase cleavage sequence added to its N-terminal end and a cysteine residue added to its C-terminal end to repetitively link, is cleaved by enterokinase on the N-terminal side of each GPR8 ligand and is subjected to a cleavage reaction to cleave on the N-terminal side of each of the C-terminal cysteine residues;
(12) The process according to (10) or (11) above, wherein the GPR8 ligand is a polypeptide that contains an amino acid sequence identical or substantially identical to the amino acid sequence of SEQ ID NO: 44;
(13) The process according to (10) or (11) above, wherein the GPR8 ligand is a polypeptide having the amino acid sequence of SEQ ID NO: 44, SEQ ID NO: 45, SEQ ID NO: 46, SEQ ID NO: 47, SEQ ID NO: 48, SEQ ID NO: 49, or SEQ ID NO: 50;
(14) The process according to (10) or (11) above, wherein the GPR8 ligand is a polypeptide having the amino acid sequence of SEQ ID NO: 44;
(15) A DNA containing a DNA segment encoding a precursor protein containing repetitive links of peptides of interest, each of which has a methionine residue or a protease cleavage sequence added to its N-terminal end and a cysteine residue or a cysteinyl peptide added to its C-terminal end to repetitively link (wherein the peptide moiety of the cysteinyl peptide is a peptide different from the peptide of interest and does not contain any methionine residue when a methionine residue is added to the N-terminal end of each peptide of interest);
(16) A recombinant vector containing the DNA segment of (15) above;
(17) The recombinant vector according to (16) above, incorporated in an E. coli transformant MM294(DE3)/pTCGPR3 designated as FERM BP-8023;
(18) A transformant transformed by the recombinant vector of (16) above;
(19) The transformant according to (18) above, which is an E. coli transformant MM294(DE3)/pTCGPR3 designated as FERM BP-8023;
(20) A precursor protein or its salt containing repetitive links of peptides of interest, each of which has a methionine residue or a protease cleavage sequence added to its N-terminal end and a cysteine residue or a cysteinyl peptide added to its C-terminal end to repetitively link (wherein the peptide moiety of the cysteinyl peptide is a peptide different from the peptide of interest and does not contain any methionine residue when a methionine residue is added to the N-terminal end of each peptide of interest); and
(21) The process according to (4) above, wherein the precursor protein is a recombinant precursor protein produced by culturing the transformant of (18).

### BRIEF DESCRIPTION OF THE DRAWING

Fig. 1 is a schematic diagram illustrating a method for preparing a peptide in accordance with the present invention. As shown, the downward arrow indicates left-handed scissors A (as means to cleave on the N-terminal side and expose the N-terminal) and the upward arrow indicates right-handed scissors B (as means to cleave on the C-terminal side and expose the C-terminal). The left-handed scissors may be cyanogen bromide, factor Xa, enterokinase or other enzymes that can cleave on the C-terminal side of a cleavage site to expose a new N-terminal. Specifically, the precursor protein is cleaved on the C-terminal side of the Ile-Glu-Gly-Arg or Asp-Asp-Asp-Asp-Lys sequence to expose the N-terminal of the peptide of interest.
   Right-handed scissors on the other hand act by S-cyanylation, followed by cleavage on the N-terminal side of Cys, to expose the C-terminal of the peptide of interest.
Fig. 2 is a schematic diagram illustrating a method for preparing a peptide in accordance with the present invention.

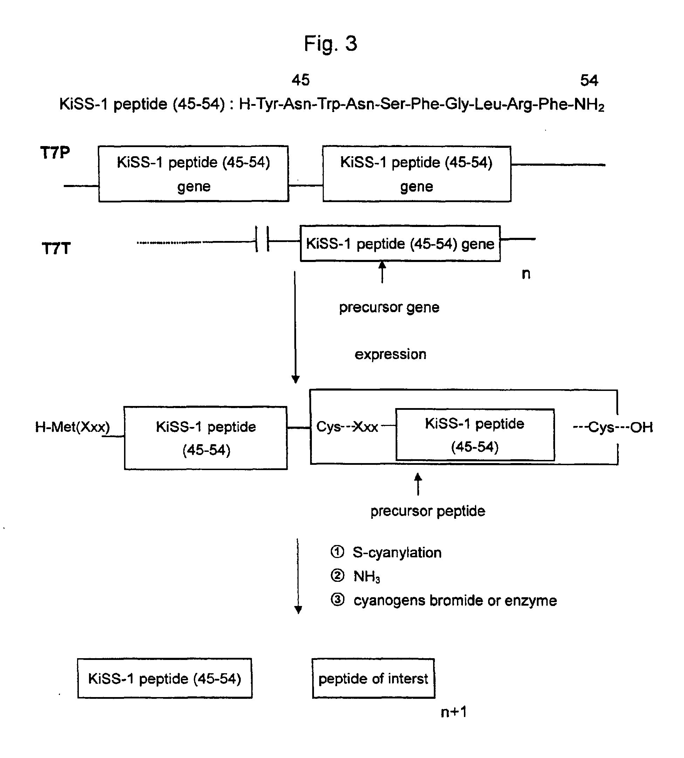

Fig. 3 is a schematic diagram illustrating a method for preparing peptide KiSS-1 in accordance with the method of the present invention.
   Xxx denotes Met (cyanogen bromide), Ile-Glu-Gly-Arg (factor Xa), Asp-Asp-Asp-Asp-Lys (enterokinase), or the like.
   Xxx is preferably a cleavage site for factor Xa or enterokinase when the peptide of interest contains any Met (which is not the case with the peptide KiSS-1 of the present invention). Other examples of Xxx include (1) a cleavage site for proline-specific endopeptidase for peptides that do not contain Pro; (2) a cleavage site for lysyl endopeptidase for peptides that do not contain Lys; (3) a cleavage site for arginylendopeptidase for peptides that do not contain Arg; (4) a cleavage site for V8 protease for peptides that do not contain Glu or Asp; (5) a cleavage site for trypsin for peptides that do not contain any basic amino acid; and (6) a cleavage site for chymotrypsin for peptides that do not contain any aromatic amino acid.

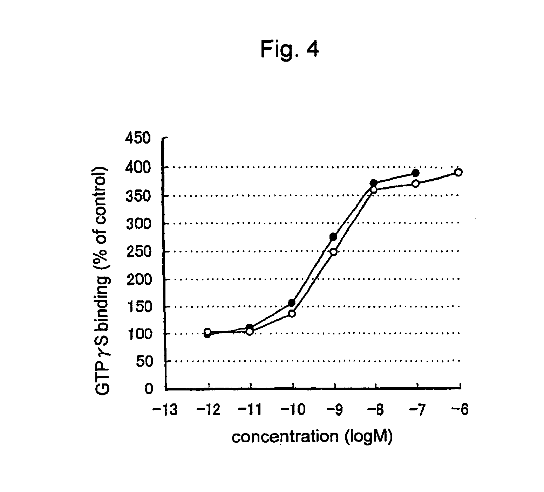

Fig. 4 is a graph showing the GTPγS binding activity of hGPR8L (SEQ ID NO: 44) of Example 1 to 3 toward CHO cells expressing GPR8. The horizontal axis indicates the concentration of hGPR8L while the vertical axis indicates the GTPγS binding activity. The solid points (-•-) indicate the results obtained for a synthetic hGPR8L (SEQ ID NO: 44) while the open points (-o-) indicate the results obtained for hGPR8L (SEQ ID NO: 44) produced by the method of the present invention (tandem repeat technique).

### MODES FOR CARRYING OUT THE INVENTION

In the method of the present invention, a peptide of interest may be any peptide that does not contain within its molecule a cleavage site recognized upon excision of the peptide. The peptide of interest may have any amino acid sequence that can be produced by gene recombination techniques.

While the peptide of interest may consist of any number of amino acid residues, it typically consists of about 10 to 100 amino acid residues, preferably about 10 to 50 amino acid residues, and more preferably about 20 to 40 amino acid residues.

Also, while the peptide of interest may have any molecular weight, it typically has a molecular weight of about 1000 to 10000 Dalton, preferably about 1000 to 5000 Dalton, and more preferably about 2000 to 4000 Dalton.

Examples of the peptide of interest include the peptide KiSS-1 (WO00/24890), RFRP-1 (WO00/29441), apelin (WO99/33976), PrRP (WO97/24436), GALP (WO99/48920), ligands for GPR8 (W 01/98494), angiotensin, bradykinin, calcitonin, conotoxin, corticotropin releasing factor, dynorphin, endorphin, encephalin, galanin, gastrin, glucagon, growth hormone releasing factor, FMRF-amide, neurokinin, neuromedin, neuropeptides, nociceptin, nocistatin, orexin-B, secretin, substance P, urocortin, VIP, PACAP, ACTH, various opioid peptides, and peptide fragments thereof. Of these peptides, the peptide KiSS-1, RFRP-1, ligand for GPR8, apelin, PrRP, and GALP are particularly preferred.

The peptide of interest is depicted according to common practice of peptide notation: the left end corresponds to the N-terminal (amino terminal) of the peptide and the right end corresponds to the C-terminal (carboxyl terminal) of the peptide.

The peptide KiSS-1 may for example be human KiSS-1 peptide described in WO 00/24890. One specific example is a peptide consisting of 8 to 54 amino acid residues and containing an amino acid sequence from amino acid 47 to 54 (with respect to the N-terminal) of the amino acid of SEQ ID NO: 1, which consists of 54 amino acid residues.

While "the peptide consisting of 8 to 54 amino acid residues and containing an amino acid sequence from amino acid 47 to 54 (with respect to the N-terminal) of the amino acid of SEQ ID NO: 1" may be any peptide that meets the requirement, each must have substantially the same activities (for example, the ability of the peptide to bind to its receptor and the ability of the peptide to stimulate the cells expressing its receptors). Specifically, the peptide may be (1) the peptide having the amino acid sequence of SEQ ID NO: 1, or (2) a peptide consisting of 8 to 15 amino acid residues and containing, at the C-terminal thereof, a sequence from amino acid 47 to 54 (with respect to the N-terminal) of the amino acid sequence of SEQ ID NO: 1.

Specific examples of KiSS-1 peptide include (1) the peptide having the amino acid sequence of SEQ ID NO: 1, (2) a peptide having a sequence from amino acid 40 to 54 (with respect to the N-terminal) of the amino acid sequence of SEQ ID NO: 1; (3) a peptide having a sequence from amino acid 45 to 54 (with respect to the N-terminal) of the amino acid sequence of SEQ ID NO: 1 (i.e., the amino acid sequence of SEQ ID NO: 2); (4) a peptide having a sequence from amino acid 46 to 54 (with respect to the N-terminal) of the amino acid sequence of SEQ ID NO: 1; (5) a peptide having a sequence from amino acid 47 to 54 (with respect to the N-terminal) of the amino acid sequence of SEQ ID NO: 1; and (6) a peptide having a sequence from amino acid 35 to 54 (with respect to the N-terminal) of the amino acid sequence of SEQ ID NO: 1.

The above-described peptides KiSS-1 each act as a ligand for the receptor protein OT7T175 described in WO 00/24890.

Examples of RFRP-1 include Rf amide-related peptides (See, Hinuma *et al*., Nature Cell Biology. 2 (2000); 703-708) and the polypeptides described in WO 00/29441. Specifically, RFRP-1 may be any peptide that has an amino acid sequence of SEQ ID NO: 1 or SEQ ID NO: 9 and act as a ligand for the receptor OT7T022 (See, Hinuma *et al*., Nature Cell Biology. 2 (2000): 703-708, and WO 00/29441).

More specifically, RFRP-1 may be, for example, a polypeptide having an amino acid sequence of SEQ ID NO: 6, SEQ ID NO: 7, SEQ ID NO: 8, SEQ ID NO: 9, SEQ ID NO: 10, or SEQ ID NO: 11

Examples of apelin include apelin-36 (a polypeptide having an amino acid sequence of SEQ ID NO: 18), apelin-13 (a polypeptide having a sequence from amino acid 24 to 36 of the amino acid sequence of SEQ ID NO: 18), and a peptide obtained by converting the N-terminal amino acid of apelin-13 (Gln) into pyroglutamic acid (See, Biochem. Biophys, Res. Commun. 251 (1998): 471-476). Apelin may be any peptide that acts as a ligand for apelin receptor, APJ (See, O'Dowd, B.F., *et al*., Gene 436 (1993): 355-359). One specific example is a polypeptide capable of binding receptor proteins that have an amino acid sequence identical or substantially identical to the amino acid sequence of SEQ ID NO. 3 described in WO 99/33976.

Examples of PrRP (19P2 ligand) include the peptides described in WO 97/24436. Specific examples include peptides that are identical or substantially identical to bovine 19P2L (b19P2L or bovine PrRP) (SEQ ID NO: 20), rat 19P2L9 (r19P2L or rat PrRP) (SEQ ID NO: 21), or human 19P2L (h19P2L or human PrRP) (SEQ ID NO: 22), and amides, esters, and salts thereof.

As used herein, the term "qualitatively substantially identical" means that the peptides are qualitatively substantially identical to one another. For example, peptides are qualitatively substantially identical if they can bind to the same type of receptors. Thus, qualitatively substantially identical peptides may still quantitatively differ from one another and the quantitative nature of the peptides, such as binding affinity of the peptides to a receptor or molecular weight of the peptide, may vary from one peptide to another.

Aside from the peptides containing the amino acid sequence of SEQ ID NO: 20, SEQ ID NO: 21 or SEQ ID NO: 22, examples of PrRP further include peptides that have about 50 to 99.9% homology, preferably 70 to 99.9% homology, more preferably 80 to 99.9% homology, and still more preferably 90 to 99.9% homology, to one of the amino acid sequences of SEQ ID NO: 20, SEQ ID NO: 21 and SEQ ID NO: 22 and have an activity qualitatively substantially identical to that of one of the peptides containing the amino acid sequence of SEQ ID NO: 20, SEQ ID NO: 21 or SEQ ID NO: 22 (it should be noted that these peptides do not contain cysteine residue in their amino acid sequences).

More specific examples of the peptides substantially identical to any of the peptides containing the amino acid sequence of SEQ ID NO: 20, SEQ ID NO: 21 or SEQ ID NO: 22 include peptides containing the peptide fragment of SEQ ID NO: 23 (In SEQ ID NO: 23, Xaa corresponding to the amino acid 10 is Ala or Thr; Xaa corresponding to the amino acid 11 is Gly or Ser, and Xaa corresponding to the amino acid 21 is OH, Gly, or Gly-Arg).

Even more specific examples are peptides containing amino acid sequences resulting from either deletion of 1 to 15 amino acids, preferably 1 to 10 amino acids, and more preferably 1 to 5 amino acids, from the amino acid sequence of SEQ ID NO: 20, SEQ ID NO: 21 or SEQ ID NO: 22, or addition of 1 to 80 amino acids, preferably 1 to 50 amino acids, and more preferably 1 to 10 amino acids, to the amino acid sequence of SEQ ID NO: 20, SEQ ID NO: 21 or SEQ ID NO: 22, or substitution of 1 to 15 amino acids, preferably 1 to 10 amino acids, and more preferably 1 to 5 amino acids, in the amino acid sequence of SEQ ID NO: 20, SEQ ID NO: 21 or SEQ ID NO: 22.

Still more specific examples are peptides containing one of the amino acid sequences of SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, and SEQ ID NO: 43.

PrRPs for use in the present invention further include those resulting from the in vivo cleavage at the N-terminal side of Gln, followed by the conversion of the terminal Gln into pyroglutamic acid.

GALP for use in the present invention may be the peptides described in WO 99/48920.

Ligands for GPR8 for use in the present invention may be any peptide that exhibits some ligand activity for GPR8, a seven-transmembrane receptor protein (O'Dowd B. F., *et al*., Genomics. 28 (1995): 84-91), such as the binding activity to GPR8 and the cell stimulatory activity against cells expressing GPR8 (*e.g.*, the ability to promote release of arachidonic acid, release of acetylcholine, release of intracellular Ca²⁺, production of intracellular cAMP, production of intracellular cGMP, production of inositol phosphate, variation of cellular membrane potential, phosphorylation of intracellular proteins, activation of c-fos, lowering pH, and GTPγS binding activity). For example, the ligands for GPR8 may be the peptides described in WO 01-98494.

More specific examples of the ligand polypeptides for GPR8 are polypeptides containing an amino acid sequence identical or substantially identical to the amino acid sequence of SEQ ID NO: 44.

Examples of the amino acid sequences substantially identical to the amino acid sequence of SEQ ID 44 include the amino acid sequences of SEQ ID 45, SEQ ID 46, SEQ ID 47, SEQ ID 48, SEQ ID 49, and SEQ ID 50.

Thus, among specific examples of the polypeptides containing an amino acid sequence identical or substantially identical to the amino acid sequence of SEQ ID NO: 44 are:
(1) a GPR8 ligand having the amino acid sequence of SEQ ID 44;
(2) a GPR8 ligand having the amino acid sequence of SEQ ID 45;
(3) a GPR8 ligand having the amino acid sequence of SEQ ID 46;
(4) a GPR8 ligand having the amino acid sequence of SEQ ID 47;
(5) a GPR8 ligand having the amino acid sequence of SEQ ID 48;
(6) a GPR8 ligand having the amino acid sequence of SEQ ID 49; and
(7) a GPR8 ligand having the amino acid sequence of SEQ ID 50.

Examples of salts of the peptides of interest include salts formed with physiologically acceptable bases (*e.g.*, alkali metals) and acids (organic and inorganic acids). Of these salts, physiologically acceptable acid addition salts are particularly preferred. Example of the salts include salts formed with an inorganic acid (for example, hydrochloric acid, phosphoric acid, hydrobromic acid, and sulfuric acid) and salts formed with an organic acid (for example, acetic acid, formic acid, propionic acid, fumaric acid, maleic acid, succinic acid, tartaric acid, citric acid, malic acid, oxalic acid, benzoic acid, methansulfonic acid, and benzenesulfonic acid).

Examples of enzymatic cleavage sites that can be added to the N-terminals of the peptides of interest based on a production process A of the present invention include:
(1) Asp-Asp-Asp-Asp-Lys (SEQ ID NO: 58) (encoded by DNA having the base sequence: GATGACGACGACAAG (SEQ ID NO: 59)), which serves as a cleavage site for a protease enterokinase;
(2) Ile-Glu-Gly-Arg (SEQ ID NO: 60) (encoded by DNA having the base sequence: ATTGAAGGCCGC (SEQ ID NO: 61)), which serves as a cleavage site for a protease factor Xa; and
(3) Gly-Pro-Arg (SEQ ID NO: 62) (encoded by DNA having the base sequence: GGCCCGCGC(SEQ ID NO: 63)), which serves as a cleavage site for a protease thrombin.

Examples of chemical cleavage sites that can be added to the N-terminals of the peptides of interest include methionine residue, which can be cleaved by cyanogen bromide.

Examples of chemical cleavage sites that can be added to the C-terminals of the peptides of interest include cysteine residue and cysteinyl peptides.

The peptide moiety of the cysteinyl peptide consists of one or more amino acid residues (for example, 1 to about 5 amino acid residues). Preferred amino acid residues, though not limited to particular types, include Gly, Ala, Ser, and Leu. Also, the peptide moiety of the cysteinyl peptide is different from the peptide of interest. Furthermore, when a methionine residue is added to the N-terminal of the peptide of interest (by using cyanogen bromide), the peptide moiety of the cysteinyl peptide does not contain a methionine residue.

By saying "precursor proteins (including precursor peptides) formed by repetitively linking peptides of interest, each with an enzymatic or chemical cleavage site added to its N-terminal and C-terminal," it is intended to refer to proteins in which two or more peptides (for example, 2 to 100, preferably 2 to about 20, and more preferably 2 to about 10 peptides), each with the above-described enzymatic or chemical cleavage site added to its N-terminal and C-terminal, are repeatedly linked.

Regarding a production process B of the present invention, by saying "precursor proteins (including precursor peptides) formed by repetitively linking peptides of interest, each with a methionine residue or a protease cleavage site added to its N-terminal and a cysteine residue or a cysteinyl peptide added to its C-terminal (with the proviso that the peptide moiety of the cysteinyl peptide differs from any of the peptides of interest and the peptide moiety does not contain any methionine residue when a methionine residue is added to the N-terminal (by using cyanogen bromide))," it is intended to encompass precursor proteins in which 2 or more of the above-described proteins (for example, 2 to 100, preferably 2 to about 20, and more preferably 2 to about 10) are repeatedly linked, with each having a methionine residue or a protease cleavage site added to its N-terminal and a cysteine residue or a cysteinyl peptide added to its C-terminal and an optional amino acid residue further added to the terminal.

The protease cleavage sequences and the cysteinyl peptides are as described above.

Preferably, the precursor proteins for use in the production method A or B of the present invention are genetically engineered recombinant precursor proteins.

Such recombinant precursor proteins may be produced, for example, by culturing a transformant containing a vector having DNA encoding the precursor protein to allow the transformant to express the precursor protein.

The DNA encoding the precursor protein may be any DNA that codes for the above-described precursor protein and its entire base sequence may be chemically synthesized. This may be done by using phosphoramidite technique, phosphortriester technique, diester technique, and hydrogen phosphonate technique and other known techniques. Short DNA strands can be synthesized at once whereas long strands are obtained by first individually synthesizing short strands and then ligating them using T4 DNA ligase.

The DNA for encoding the peptides of interest that is used in the production of DNA encoding the precursor protein may be known DNA or DNA cloned using a known technique. As long as the DNA sequence is coding for the peptides of interest, the sequence may be either a naturally occurring base sequence or a base sequence obtained by substituting a codon of a naturally occurring base sequence with another codon for the same amino acid.

The DNA encoding a precursor protein in which peptides of interest, each with an enzymatic or chemical cleavage site added to its N-terminal and C-terminal, are repetitively linked is constructed as the repetitive sequence by flanking each DNA base sequence encoding each of the peptides of interest with base sequences encoding an enzymatic or chemical cleavage site (*i.e.*, the sequence containing the cleavage site is introduced at the 5'-end side and the 3'-end side of each peptide-coding sequence).

The DNA encoding a precursor protein in which peptides of interest, each with a methionine residue or a protease cleavage sequence added to its N-terminal and a cysteine residue or a cysteinyl peptide added to its C-terminal, are repetitively linked is constructed as the repetitive sequence by introducing a base sequence encoding a cleavage site for cyanogen bromide or a protease (to cleave a peptide bond on the N-terminal side) and a base sequence encoding a cleavage site that, after subjected to the S-cyanylation using a 1-cyano-4-(dimethylamino) pyridium salt (DMAP-CN), can be cleaved by hydrolysis or ammonolysis (e.g., TGT or TGC each coding for cysteine, and base sequences coding for cysteinyl peptides) at the 5'-end and at the 3'-end of each DNA base sequence encoding each of the peptides of interest, respectively.

The C-terminal of the precursor protein may be a cysteine residue or a cysteinyl peptide.

While the DNA encoding KiSS-1 peptide may be any DNA that encodes any of the above-described KiSS-1 peptides, (1) DNA having the base sequence of SEQ ID NO: 3 may be used as the DNA encoding human KiSS-1 peptide having the amino acid sequence of SEQ ID NO: 1, and (2) DNA having the base sequence of SEQ ID NO: 4 may be used as the DNA encoding KiSS-1 peptide (45-54) having the amino acid sequence of SEQ ID NO: 2, for example. Codons in these DNA may be substituted for other codons for the same amino acids.

One specific example of the DNA containing the DNA segment encoding KiSS-1 peptide for use in the method of the present invention is a DNA containing the following base sequence (SEQ ID NO: 5): 
GGTAGCGCGA TGTATAACTG GAACAGCTTT GGTCTGCGTT TTTGTGGCTC

GGCGATGTAC AATTGGAATT CCTTCGGCCT GCGCTTCTGC GGCTCGGCGA

TGTATAACTG GAACTCCTTT GGCCTGCGCT TTTGCGGTTC TGCT
 This sequence is obtained by linking a base sequence encoding a cleavage site for cyanogen bromide (ATG) and a base sequence encoding a cleavage site for a 1-cyano-4-(dimethylamino) pyridium salt (DMAP-CN) (TGT) to the 5'-end and the 3'-end, respectively, of the base sequence of SEQ ID NO: 4, which encodes KiSS-1 peptide (45-54). By linking a series of this base sequence in a repetitive manner, a DNA segment encoding the precursor protein of KiSS-1 peptide can be produced.

While the DNA encoding RFRP-1 peptide may be any DNA that encodes any of the above-described RFRP-1 peptides, for example, (1) DNA having the base sequence of SEQ ID NO: 12 may be used as the DNA encoding RFRP-1 having the amino acid sequence of SEQ ID NO: 6; (2) DNA having the base sequence of SEQ ID NO: 13 may be used as the DNA encoding RFRP-1 having the amino acid sequence of SEQ ID NO: 7; (3) DNA having the base sequence of SEQ ID NO: 14 may be used as the DNA encoding RFRP-1 having the amino acid sequence of SEQ ID NO: 8; (4) DNA having the base sequence of SEQ ID NO: 15 may be used as the DNA encoding RFRP-1 having the amino acid sequence of SEQ ID NO: 9; (5) DNA having the base sequence of SEQ ID NO: 16 may be used as the DNA encoding RFRP-1 having the amino acid sequence of SEQ ID NO: 10; and (5) DNA having the base sequence of SEQ ID NO: 17 may be used as the DNA encoding RFRP-1 having the amino acid sequence of SEQ ID NO: 11.

While the DNA encoding apelin may be any DNA that encodes any of the above-described apelins, for example, (1) DNA having the base sequence of SEQ ID NO: 19 may be used as the DNA encoding apelin-18 having the amino acid sequence of SEQ ID NO: 18.

While the DNA encoding PrRP may be any DNA that encodes any of the above-described PrRPs, the DNA described in WO 97-24436 can be specifically used.

While the DNA encoding GALP may be any DNA that encodes any of the above-described GALPs, the DNA described in WO 99/48920 can be specifically used.

While the DNA encoding a ligand for GPR8 may be any DNA that encodes any of the above-described ligands for GALP, for example, (1) DNA having the base sequence of SEQ ID NO: 51 may be used as the DNA encoding the GPR8 ligand having the amino acid sequence of SEQ ID NO: 44; (2) DNA having the base sequence of SEQ ID NO: 52 may be used as the DNA encoding the GPR8 ligand having the amino acid sequence of SEQ ID NO: 45; (3) DNA having the base sequence of SEQ ID NO: 53 may be used as the DNA encoding the GPR8 ligand having the amino acid sequence of SEQ ID NO: 46; (4) DNA having the base sequence of SEQ ID NO: 54 may be used as the DNA encoding the GPR8 ligand having the amino acid sequence of SEQ ID NO: 47; (5) DNA having the base sequence of SEQ ID NO: 55 may be used as the DNA encoding the GPR8 ligand having the amino acid sequence of SEQ ID NO: 48; (6) DNA having the base sequence of SEQ ID NO: 56 may be used as the DNA encoding the GPR8 ligand having the amino acid sequence of SEQ ID NO: 49; and (7) DNA having the base sequence of SEQ ID NO: 57 may be used as the DNA encoding the GPR8 ligand having the amino acid sequence of SEQ ID NO: 50.

One specific example of the DNA encoding a ligand for GPR8 for use in the method of the present invention is the DNA having the following base sequence (SEQ ID: 64): 
GATGACGATG ACAAATGGTA TAAACATGTG GCGAGCCCGC GTTATCATAC
CGTGGGCCGC GCGGCCGGTC TGCTGATGGG CCTGTGTCAA TTGGGTGGTG
ATGACGATGA CAAATGGTAT AAACATGTGG CGAGCCCGCG TTATCATACC
GTGGGCCGCG CGGCCGGTCT GCTGATGGGC CTGTGTGAGC TCGGCTCTGA
CGACGATGAT AAATGGTACA AACACGTTGC CTCCCCGCGC TACCACACGG
TTGGTCGTGC CGCGGGCCTG CTGATGGGTC TGTGCGGT
 This base sequence is obtained by linking a base sequence (SEQ ID NO: 59) encoding an enterokinase cleavage sequence (Asp-Asp-Asp-Asp-Lys) (SEQ ID NO: 58) and a base sequence encoding a cleavage site for a 1-cyano-4-(dimethylamino) pyridium salt (DMAP-CN) (TGT) to the 5'-end and the 3'-end, respectively, of the base sequence of SEQ ID NO: 44, which encodes a ligand for GRP8 (23 residues). By linking a series of this base sequence in a repetitive manner, a DNA segment encoding the precursor protein of ligand for GRP8 (23 residues) can be produced.

The DNA encoding the precursor protein, in which the peptides of interest are repetitively linked, may be altered to a DNA encoding muteins of the peptides of interest by using a conventional genetic engineering technique, such as site-specific mutagenesis.

Site-specific mutagenesis is a known technology and is described in many literatures (See, Lather, R. F.; Lecoq, J. P. Genetic Engineering. Academic Press; 1983: 31-50), and so is oligonucleotide-directed mutagenesis (See, Smith, M.; Gillam, S. Genetic Engineering: Principles and Methods. Plenum Press; 3 1981: 1-32).

Among examples of plasmids to serve as the vector incorporating the DNA encoding the precursor protein are pBR322 (Gene. 2 (1977): 95), pBR313 (Gene. 2 (1977): 75), pBR324, pBR325 (Gene. 4 (1978): 124), pBR327, pBR328 (Gene. 9 (1980): 287), pBR329 (Gene. 17 (1982): 79), pKY2289 (Gene. 3 (1978): 1), pKY2700 (SEIKAGAKU. 52 (1980): 770), pACYC177, pACYC184 (Journal of Bacteriology. 134 (1978): 1141), pRK248, pRK646, pDF (Methods in Enzymology. 68 (1979): 268), and pUC18 and pUC19 (Yanisch-Perron *et al*. Gene. 33 (1985): 103), each derived from Escherichia coli.

Other examples of the vectors include bacterio phages such as λ-phage vectors of λgt system, including λgt·λC (Proc. Nat. Acad, Sci. U.S.A. 71 (1974): 4579), ·t·λB (Proc. Nat. Acad, Sci. U.S.A. 72 (1975): 3461) and λDam (Gene. 1 (1977): 255), Charon vectors (Science. 196 (1977): 161; Journal of Virology. 29 (1979): 555), and filamentous phage-vectors of mp system, such as mp18 and mp19 (Yanisch-Perron *et al*. Gene. 33 (1985): 103).

Preferably, these DNA constructs have a promoter upstream of ATG. The promoter may be any promoter that is suitable for use with the hosts used to create transformants. For example, when E. coli is used as the host, promoters such as trp promoter, lac promoter, recA promoter, λPL promoter, lpp promoter, and T7 promoter may be used.

When it is desired to use the T7 promoter system, ϕ10 promoter is preferably used (Rosenberg A. H. *et al*. Gene. 56 (1987): 125-135) while any of the 17 different promoters found on the T7 DNA may also be used (J. L. Oakley *et al*. Proc. Natl. Acad. Sci. U.S.A. 74 (1977): 4266-4270; Rosa M. D. Cell. 16 (1979): 815-825; Panayotatos N. *et al*. Nature. 280 (1979): 35; and Dunn J. J. *et al*. J. Mol. Biol. 166 (1983): 477-535).

While any transcription terminator operable in E. coli system may be used, Tϕ terminator is preferably used (Studier F. W. et al. J. Mol. Biol. 189 (1986): 113-130).

T7 RNA polymerase DNA for use in the present invention may be T7 DNA (Studier F.W. *et al*. J. Mol. Biol. 189 (1986): 113-130).

Preferably, vectors for use in the present invention are constructed with T7 promoter and T7 terminator. Examples of such vectors include pET-1, pET-2, pET-3, pET-4 and pET-5 (Rosenberg, A. H. Gene. 56 (1987): 125-135), and pTB960-2 (EP-A-499990). Of these, pTB960-2 is particularly preferred.

Transformants for use in the present invention can be created by using a known technique to transfect a host with the expression plasmid obtained by the above-described techniques (See, for example, Cohen S.N. Pro. Natl. Acad. Sci. U.S.A. 69 (1972): 2110).

Microorganisms for use in the present invention that are used as hosts to create the transformants may be bacteria of the genus *Escherichia.*

Examples of the members of the genus Escherichia used for this purpose include E. coli. Specifically, E. coli strains, such as K12DH1 (Pro. Natl. Acad. Sci. U.S.A. 60 (1968): 160), JM-103 (Nucleic Acids Research. 9 (1981): 309), JA221 (Journal of Molecular Biology. 120 (1978): 517), HB101 (Journal of Molecular Biology. 41 (1969): 459), C600 (Genetics. 39 (1954): 440), N4830 (Cell. 25 (1981): 713), K-12 MM294 (Pro. Natl. Acad. Sci. U.S.A. 73 (1976): 4174), and BL-21, may be used.

When it is desired to use the T7 promoter system to create transformants, the host cells may be E. coli strains (*e.g.*, MM294, DH-1, C600, JM109, and BL21) into which T7 RNA polymerase DNA (T7DNA1) has been integrated (Studier F.W. *et al*. J. Mol. Biol. 189 (1986): 113-130), and E. coli strains into which T7 RNA polymerase DNA (T7DNA1) has been introduced along with other plasmids. Among preferred hosts are MM294 and BL21 strains lysogenized with λ-phage into which T7DNA1 has been integrated. The promoter for the expression of T7DNA1 may be lac promoter that can be induced by isopropyl-1-thio-β-D-galactopyranoside (also referred to as IPTG).

The recombinant precursor proteins for use in the present invention can be prepared by culturing the above-described transformant in a culture medium and collecting the recombinant precursor protein produced by the transformant.

Preferably, the culture medium has a pH of about 6 to 8. One preferred example of the culture medium for culturing bacteria of the genus *Escherichia* is the M9 medium containing, for example, glucose and casamino acid (Miller Journal of Experiments in Molecular Genetics. Cold Spring Harbor Laboratory, New York (1972): 431-433). When necessary, agents such as 3β-indolyl acrylic acid and isopropyl-β-D-thiogalactopyranoside may be added to facilitate the activity of the promoter.

When bacteria of the genus *Escherichia* are used as the host, the bacteria are generally cultured at about 15 to 43°C for about 3 to 24 hours. Ventilation and stirring may be provided as required.

In cases where T7 promoter system is employed, either
(1) IPTG is added to allow T7DNA (RNA polymerase DNA) connected downstream of the lac promoter to be expressed, or
(2) the temperature of the culture is increased to allow T7DNA (RNA polymerase DNA) connected downstream of the λPL promoter to be expressed. In each case, the resulting T7 phage RNA polymerase 1 specifically allows the operation of the T7 promoter.

After culturing, the cells are collected using a known technique, are suspended for example in a buffer solution, and are then lysed for example by treatment with a protein-denaturing agent, sonication, treatment with an enzyme such as lysozyme, processing with glass beads or a French press, and freeze-thaw process. Using a known technique such as centrifugation, the precursor protein is then collected either in the form of inclusion-body or in a soluble fraction (supernatant). Inclusion-body is preferred to a soluble fraction.

The inclusion-bodies so obtained may be solubilized with a denaturing agent before subjected to the subsequent processes. To isolate the recombinant precursor protein from the supernatant, common protein purification techniques can be used. For example, purification techniques such as gel filtration, ion-exchange chromatography, adsorption chromatography, high-performance liquid chromatography, affinity chromatography, hydrophobic chromatography, and electrophoresis can be used in a suitable combination. Alternatively, the precursor protein may be subjected to the subsequent processes without any purification or in a partially purified form.

Using cyanogen bromide, a protease or the like, the recombinant precursor protein so obtained is then cleaved at the N-terminal side of each of the peptides of interest.

The protease use for this purpose may be any known protease and may preferably be for example enterokinase, factor Xa, or thrombin. Of these, enterokinase and factor Xa are particularly preferred.

The protease is typically used in an amount of about 0.01 units to about 100 units, and preferably about 0.1 units to about 10 units, with respect to 1mg of the recombinant polypeptide.

When cyanogen bromide is used in the cleavage reaction, a methionine residue to serve as a cleavage site for cyanogen bromide is added to the N-terminal of each of the peptides of interest in the recombinant precursor protein. In such a case, it is preferred that none of the peptides of interest contain a methionine residue.

When enterokinase is used as the protease, a sequence that serves as a cleavage site for enterokinase (Asp-Asp-Asp-Asp-Lys; SEQ ID NO: 54) is ligated to the N-terminal of each of the peptides of interest in the recombinant precursor protein. In such a case, it is preferred that none of the peptides of interest contain the sequence Asp-Asp-Asp-Asp-Lys or the like.

When factor Xa is used to serve as the protease, a sequence that serves as a cleavage site for factor Xa (Ile-Glu-Gly-Arg; SEQ ID NO: 56) is ligated to the N-terminal of each of the peptides of interest in the recombinant precursor protein. In such a case, it is preferred that none of the peptides of interest contain the sequence Ile-Glu-Gly-Arg or the like.

When thrombin is used to serve as the protease, a sequence that serves as a cleavage site for thrombin (Gly-Pro-Arg; SEQ ID NO: 58) is ligated to the N-terminal of each of the peptides of interest in the recombinant precursor protein. In such a case, it is preferred that none of the peptides of interest contain the sequence Gly-Pro-Arg or the like.

The cleavage of peptide bonds by the protease is typically carried out at a temperature in the range of about 0°C to about 60°C and preferably in the range of 0°C to about 40°C.

While the reaction may be carried out in any suitable solvent, solvents such as Tris-HCl buffer, Tris-acetic acid buffer, phosphate-buffered solution, and borate-buffered solution are preferred.

The reaction is typically carried out at a pH of about 1 to about 12, and preferably at a pH of about 4 to about 8.

The cleavage of the recombinant precursor protein at the C-terminal side of each of the peptides of interest can be carried out according to the method described in EP887417. Specifically, the recombinant precursor protein is cleaved at the N-terminal side of a cysteine that is located on the C-terminal side of each of the peptides of interest.

The cleavage may be carried out by S-cyanylation reaction followed by hydrolysis for example. When it is desired to obtain final products in the form of amides or salts of the desired peptides, the cleavage may be carried out by S-cyanylation reaction followed by ammonolysis for example. To carry out the S-cyanylation reaction, an S-cyanylating reagent is reacted with the reactant.

Examples of the S-cyanylating reagent include 2-nitro-5-thiocyanobenzoic acid (NTCB), 1-cyano-4-(dimethylamino) pyridium salt (DMAP-CN), and CN⁻ ion.

The S-cyanylating reagent is generally used in an amount of about 2 times to 50 times, preferably about 5 times to 10 times, the amount of the total thiol groups as measured in the number of moles.

The reaction may be carried out at any temperature in the range of about 0°C to about 80°C and is preferably carried out at a temperature of about 0°C to about 50°C. The solvent used may be any solvent that does not react with the S-cyanylating reagent and may preferably be Tris-HCl buffer, Tris-acetic acid buffer, phosphate-buffered solution, or borate-buffered solution. An organic solvent that does not react with the S-cyanylating reagent may also be present.

The reaction may be carried out at a pH of 1 to 12. Specifically, the reaction is preferably carried out in a pH range of 7 to 10 when NTCB is used, whereas when DMAP-CN is used, the reaction is preferably carried out in a pH range of 2 to 7 to prevent S-S exchange reaction. A denaturing agent such as guanidine hydrochloride may be added to the reaction mixture.

The above-described ammonolysis or hydrolysis may be carried out by treating the reactant with an alkali.

The alkali treatment may be provided by adjusting the pH of the aqueous solution of the reactant to a pH of 7 to 14.

Specifically, the pH of the solution may be adjusted by adding to the aqueous solution of the reactant a proper amount of a solution of, for example, ammonia, sodium hydroxide, an amino compound, Trizma Base (trade name) (Tris(hydroxymethy)-aminomethane), disodium hydrogenphosphate, potassium hydroxide, or barium hydroxide. Of these, ammonia is particularly preferred.

The concentration of the solution is from about 0.01 to 15N, and preferably from about 0.1 to 3N for ammonia and amino compounds; is from about 0.01 to 2N and preferably from about 0.05 to 1N for sodium hydroxide; is from about 1mM to 1M and preferably from about 20mM to 200mM for Trizma Base (trade name); is from about 1mM to 1M and preferably from about 10mM to 100mM for disodium hydrogenphosphate; and is from about 0.01 to 4N and preferably from about 0.1 to 2N for potassium hydroxide. The reaction may be carried out at any temperature of about -20°C to 80°C and is preferably carried out at a temperature of about -10°C to 50°C.

As for the reaction time, the S-cyanylation reaction is carried out over a time period of about 1 to 60 minutes, preferably about 15 to 30 minutes; the hydrolysis is carried out over a time of about 5 minutes to 100 hours, preferably about 10 minutes to 15 hours; and the ammonolysis is carried out over a time period of about 5 minutes to 24 hours, preferably about 10 to 180 minutes.

The amino compound may be a compound represented by, for example, the following chemical formula: R¹-(NR²)-H (wherein R¹ and R² are each independently (i) hydrogen, (ii) C₁₋₂₀ alkyl, C₃₋ ₈ cycloalkyl, C₆₋₁₄ aryl, or C₆₋₁₄ aryl-C₁₋₃ alkyl (each of which may be unsubstituted or substituted with 1 to 3 amino groups or hydroxyl groups), (iii) substituted or unsubstituted amino, or (iv) hydroxyl or C₁₋₆ alkoxyl).

The peptides of interest that are excised in the cleavage reaction may be isolated by common peptide purification processes. For example, purification techniques such as gel filtration, ion-exchange chromatography, high-performance liquid chromatography, affinity chromatography, hydrophobic chromatography, thin layer chromatography, and electrophoresis can be used in a suitable combination.

When necessary, the peptides of interest may be freeze-dried and formed into powder. Upon freeze-drying, a stabilizing agent, such as sorbitol, mannitol, dextrose, maltose, trehalose, and glycerol, may be added.

The C-terminal of each peptide of interest obtained by the method of the present invention may be provided in the form of an amide (-CONH₂), carboxyl group (-COOH), carboxylate (-COO⁻), alkylamide (-CONHR), or an ester (-COOR). Examples of R in the ester or alkylamide form of the peptide include C₁₋₆ alkyl groups, such as methyl, ethyl, n-propyl, isopropyl or n-butyl; C₃₋₈ cycloalkyl groups, such as cyclopentyl and cyclohexyl; C₆₋₁₂ aryl groups, such as phenyl and α-naphthyl; phenyl-C₁₋₂ alkyl groups, such as benzyl, phenethyl and benzhydryl; or C₇₋₁₄ aralkyl groups, such as α-naphthyl-C₁₋₂ alkyls including α-naphthylmethyl; as well as pivaloyloxymethyl group, which gives an ester form widely used in oral application.

The peptides of interest, amides, esters, and salts thereof obtained by the method of the present invention may be mixed with sterile water, human serum albumin (HSA), saline, and other known physiologically acceptable carriers to make a safe pharmaceutical that can be parenterally or topically administered to mammals (*e.g*., human, primates, cows, horses, sheep, swine, rats, mice, and guinea pigs). For parenteral administration, the peptides of interest of the present invention, amides, esters, and salts thereof may be intravenously or intramuscularly administered at a dose of about 0.01mg to about 50mg, and preferably about 0.1mg to about 10mg per day per patient.

A formulation formed with the peptide of the present invention may further contain a salt, diluent, adjuvant, other carriers, buffer, binder, surfactant, preservative, and other pharmaceutically acceptable active ingredients. A parenteral formulation can be prepared either as an ampoule of a suspension formed with sterile water or other physiologically acceptable solvents or as an ampoule of sterile powder (typically obtained by freeze-drying the peptide solution) that is diluted with a physiologically acceptable diluent upon use.

Throughout the description and the drawings, amino acids, peptides, protective groups, active groups and other chemical entities may be denoted by symbols, abbreviations or codes as defined by IUPAC-IUB (Commission of Biochemical Nomenclature). The followings are some examples. Unless otherwise specified, amino acids for which optical isomers exist are all L-forms.
- DNA:: deoxyribonucleic acid
- A:: adenine
- T :: thymine
- G :: guanine
- C :: cytosine
- RNA :: ribonucleic acid
- EDTA :: ethylenediamine triacetate
- Gly :: glycine
- Ala :: alanine
- Val :: valine
- Leu :: leucine
- Ile :: isoleucine
- Ser :: serine
- Thr :: threonine
- Met :: methionine
- Glu :: glutamic acid
- Asp :: aspartic acid
- Lys :: lysine
- Arg :: arginine
- His :: histidine
- Phe :: phenylalanine
- Tyr :: tyrosine
- Trp :: tryptophan
- Pro :: proline
- Asn :: asparagine
- Gln :: glutamine
- ATP :: adenosine triphosphate
- T7P :: T7 promoter
- T7T :: T7 terminator

The followings are sequences represented by SEQ ID NOs in the present specification:

### [SEQ ID NO: 1]

Amino acid sequence of human peptide KiSS-1

### [SEQ ID NO: 2]

Amino acid sequence of human peptide KiSS-1 (45-54)

### [SEQ ID NO: 3]

DNA base sequence encoding human peptide KiSS-1 of SEQ ID NO: 1

### [SEQ ID NO: 4]

DNA base sequence encoding human peptide KiSS-1 (45-54) of SEQ ID NO: 2

### [SEQ ID NO: 5]

DNA base sequence containing DNA segment encoding a precursor protein of human peptide KiSS-1 (45-54)

### [SEQ ID NO: 6]

Amino acid sequence of RFRP-1 consisting of 9 amino acid residues

### [SEQ ID NO: 7]

Amino acid sequence of RFRP-1 consisting of 12 amino acid residues

### [SEQ ID NO: 8]

Amino acid sequence of RFRP-1 consisting of 20 amino acid residues

### [SEQ ID NO: 9]

Amino acid sequence of RFRP-1 consisting of 37 amino acid residues

### [SEQ ID NO: 10]

Amino acid sequence of RFRP-1 consisting of 9 amino acid residues

### [SEQ ID NO: 11]

Amino acid sequence of RFRP-1 consisting of 17 amino acid residues

### [SEQ ID NO: 12]

DNA base sequence encoding polypeptide of SEQ ID NO: 6

### [SEQ ID NO: 13]

DNA base sequence encoding polypeptide of SEQ ID NO: 7

### [SEQ ID NO: 14]

DNA base sequence encoding polypeptide of SEQ ID NO: 8

### [SEQ ID NO: 15]

DNA base sequence encoding polypeptide of SEQ ID NO: 9

### [SEQ ID NO: 16]

DNA base sequence encoding polypeptide of SEQ ID NO: 10

### [SEQ ID NO: 17]

DNA base sequence encoding polypeptide of SEQ ID NO: 11

### [SEQ ID NO: 18]

Amino acid sequence of apelin-36

### [SEQ ID NO: 19]

DNA base sequence encoding apelin-36

### [SEQ ID NO: 20]

Amino acid sequence of bovine PrRP

### [SEQ ID NO: 21]

Amino acid sequence of rat PrRP

### [SEQ ID NO: 22]

Amino acid sequence of human PrRP

### [SEQ ID NO: 23]

A specific example of a peptide substantially identical to the peptide of SEQ ID NO: 20, SEQ ID NO: 21, or SEQ ID NO: 22

### [SEQ ID NO: 24]

Amino acid sequence obtained by the N-terminal sequence analysis of P-3 fraction of ligand polypeptide purified from bovine hypothalamus described in WO 97/24436

### [SEQ ID NO: 25]

Amino acid sequence obtained by the N-terminal sequence analysis of P-2 fraction of ligand polypeptide purified from bovine hypothalamus described in WO 97/24436

### [SEQ ID NO: 26]

Amino acid sequence of a ligand polypeptide derived from bovine hypothalamus described in WO 97/24436

### [SEQ ID NO: 27]

Amino acid sequence of a ligand polypeptide derived from bovine hypothalamus described in WO 97/24436

### [SEQ ID NO: 28]

Amino acid sequence of a ligand polypeptide derived from bovine hypothalamus described in WO 97/24436

### [SEQ ID NO: 29]

Amino acid sequence of a ligand polypeptide derived from bovine hypothalamus described in WO 97/24436

### [SEQ ID NO: 30]

Amino acid sequence of a ligand polypeptide derived from bovine hypothalamus described in WO 97/24436

### [SEQ ID NO: 31]

Amino acid sequence of a ligand polypeptide derived from bovine hypothalamus described in WO 97/24436

### [SEQ ID NO: 32]

Amino acid sequence of a rat ligand polypeptide described in WO 97/24436

### [SEQ ID NO: 33]

Amino acid sequence of a rat ligand polypeptide described in WO 97/24436

### [SEQ ID NO: 34]

Amino acid sequence of a rat ligand polypeptide described in WO 97/24436

### [SEQ ID NO: 35]

Amino acid sequence of a rat ligand polypeptide described in WO 97/24436

### [SEQ ID NO: 36]

Amino acid sequence of a rat ligand polypeptide described in WO 97/24436

### [SEQ ID NO: 37]

Amino acid sequence of a rat ligand polypeptide described in WO 97/24436

### [SEQ ID NO: 38]

Amino acid sequence of a human ligand polypeptide described in WO 97/24436

### [SEQ ID NO: 39]

Amino acid sequence of a human ligand polypeptide described in WO 97/24436

### [SEQ ID NO: 40]

Amino acid sequence of a human ligand polypeptide described in WO 97/24436

### [SEQ ID NO: 41]

Amino acid sequence of a human ligand polypeptide described in WO 97/24436

### [SEQ ID NO: 42]

Amino acid sequence of a human ligand polypeptide described in WO 97/24436

### [SEQ ID NO: 43]

Amino acid sequence of a human ligand polypeptide described in WO 97/24436

### [SEQ ID NO: 44]

Amino acid sequence of a ligand polypeptide for GPR8 (human·1-23)

### [SEQ ID NO: 45]

Amino acid sequence of a ligand polypeptide for GPR8 (porcine·1-23)

### [SEQ ID NO: 46]

Amino acid sequence of a ligand polypeptide for GPR8 (rat·mouse·1-23)

### [SEQ ID NO: 47]

Amino acid sequence of a ligand polypeptide for GPR8 (human·1-30)

### [SEQ ID NO: 48]

Amino acid sequence of a ligand polypeptide for GPR8 (porcine·1-30)

### [SEQ ID NO: 49]

Amino acid sequence of a ligand polypeptide for GPR8 (rat·1-30)

### [SEQ ID NO: 50]

Amino acid sequence of a ligand polypeptide for GPR8 (mouse·1-30)

### [SEQ ID NO: 51]

DNA base sequence encoding a ligand polypeptide for GPR8 (human·1-23)

### [SEQ ID NO: 52]

cDNA base sequence encoding a ligand polypeptide for GPR8 (porcine·1-23)

### [SEQ ID NO: 53]

DNA base sequence encoding a ligand polypeptide for GPR8 (rat·mouse·1-23)

### [SEQ ID NO: 54]

DNA base sequence encoding a ligand polypeptide for GPR8 (human·1-30)

### [SEQ ID NO: 55]

DNA base sequence encoding a ligand polypeptide for GPR8 (porcine·1-30)

### [SEQ ID NO: 56]

DNA base sequence encoding a ligand polypeptide for GPR8 (rat·1-30)

### [SEQ ID NO: 57]

DNA base sequence encoding a ligand polypeptide for GPR8 (mouse·1-30)

### [SEQ ID NO: 58]

Amino acid sequence of a cleavage sequence for enterokinase

### [SEQ ID NO: 59]

DNA base sequence encoding a cleavage sequence for enterokinase

### [SEQ ID NO: 60]

Amino acid sequence of a cleavage sequence for factor Xa

### [SEQ ID NO: 61]

DNA base sequence encoding a cleavage sequence for factor Xa

### [SEQ ID NO: 62]

Amino acid sequence of a cleavage sequence for thrombin

### [SEQ ID NO: 63]

DNA base sequence encoding a cleavage sequence for thrombin

### [SEQ ID NO: 64]

DNA base sequence containing DNA segment encoding a precursor protein of ligand for human GPR8 (SEQ ID NO: 44)

### [SEQ ID NO: 65]

Base sequence of a DNA oligomer used to prepare a structural gene in Example 1

### [SEQ ID NO: 66]

Base sequence of a DNA oligomer used to prepare a structural gene in Example 1

### [SEQ ID NO: 67]

Base sequence of a DNA oligomer used to prepare a structural gene in Example 1

### [SEQ ID NO: 68]

Base sequence of a DNA oligomer used to prepare a structural gene in Example 1

### [SEQ ID NO: 69]

Base sequence of a DNA oligomer used to prepare a structural gene in Example 1

### [SEQ ID NO: 70]

Base sequence of a DNA oligomer used to prepare a structural gene in Example 1

### [SEQ ID NO: 71]

Base sequence of a DNA oligomer used to prepare a structural gene in Example 1

### [SEQ ID NO: 72]

Base sequence of a DNA oligomer used to prepare a structural gene in Example 1

### [SEQ ID NO: 73]

Base sequence of a DNA oligomer used to prepare a structural gene in Example 1

### [SEQ ID NO: 74]

Base sequence of a DNA oligomer used to prepare a structural gene in Example 1

The E. coli transformant MM 294 (DE3)/pTCGPR3 obtained in Example 1 below has been deposited at International Patent Organism Depository (IPOD) of National Institute of Advanced Industrial Science and Technology (AIST) of Tsukuba Central 6, 1-1-1 Higashi, Tsukuba-shi, Ibaraki-ken 305-8566, Japan, since April 17th, 2002, under Accession Number FERM BP-8023 and has also been deposited at Institute for Fermentation, Osaka (IFO) of 17-85, Juso-honmachi 2-chome, Yodogawa-ku, Osaka-shi, Osaka-fu 532-8686, Japan, since March 14th, 2002, under Accession Number IFO 16733.

### EXAMPLES

The present invention will now be described in further detail with reference to Examples, which are provided by way of example only and are not intended to limit the scope of the invention in any way.

### Example 1

### (a) Preparation of a gene encoding three tandem repeats of ligand for human GPR8 (hGPR8L; SEQ ID NO: 44)

The following 10 DNA fragments (SEQ ID NO: 65-74, in sequence listing) were used to prepare a structural gene encoding three tandem repeats of hGPR8L.
#1 
5'-TATGGATGACGATGACAAATGGTATAAACATGTGGCGAGCCCGCGTTATCATACCG

(SEQ ID NO: 65)
 #2 
5′-GCGCGGCCCACGGTATGATAACGCGGGCTCGCCACATGTTTATACCATTTGTCATCGTC
ATCCA
(SEQ ID NO: 66)
 #3 5'-TGGGCCGCGCGGCCGGTCTGCTGATGGGCCTGTGTCAATTGGGTTTGAACTTCTCTGTC 
TCCGCCGCCGGAG
(SEQ ID NO: 67)
 #4 
5′-GATCCTCCGGCGGCGGAGACAGAGAAGTTCAAACCCAATTGACACAGGCCCATCAGCAG
ACCGGCC
(SEQ ID NO: 68)
 #5 
5′-AATTGGGTGGTGATGACGATGACAAATGGTATAAACATGTGGCGAGCCCGCGTTATCAT
ACCG
(SEQ ID NO: 69)
 #6 
5′-CGGCCCACGGTATGATAACGCGGGCTCGCCACATGTTTATACCATTTGTCATCGTCATC
ACCACCC
(SEQ ID NO: 70)
 #7 
5′-TGGGCCGCGCGGCCGGTCTGCTGATGGGCCTGTGTGAGCTCGGCTCTGACGACGATGAT

AAATGGTAC

(SEQ ID NO: 71)
 #8 
5′-CAACGTGTTTGTACCATTTATCATCGTCGTCAGAGCCGAGCTCACACAGGCCCATCAGC
AGACCGGCC
(SEQ ID NO: 72)
 #9 
5′-AAACACGTTGCCTCCCCGCGCTACCACACGGTTGGTCGTGCCGCGGCCTGCTGATGGG
TCTGTGCGGTT GAG
 (SEQ ID NO: 73) #10

5′-GATCCTCAACCGCACAGACCCATCAGCAGGCCCGCGGCACGACCAACCGTGTGGTAGCG
CGGGGAGG
(SEQ ID NO: 74)

Each of DNA oligomers #2 and #3 was allowed to react in a 25µl phosphorylation reaction solution (10µg DNA oligomer, 50mM Tris-HCl, pH7.6, 10mM MgCl₂, 1mM spermidine, 10mM dithiothreitol (abbreviated as DTT, hereinafter), 0.1mg/ml bovine serum albumin (abbreviated as BSA, hereinafter), 1mM ATP, 10 units of T4 polynucleotide kinase (Takara Shuzo, Co., Ltd.)) at 37°C for 1 hour to phosphorylate 5'-end of each olygomer. After treatment with phenol, twice as much ethanol was added and the mixture was cooled to -70°C and was then centrifuged to precipitate DNA.

The resulting DNA fragments were mixed with DNA oligomers #1 and #4 to make a 120µl mixture. The mixture was then maintained at 90°C for 10 minutes and was allowed to slowly cool to room temperature for annealing. Using TaKaRa DNA ligation kit ver. 2 (Takara Shuzo, Co., Ltd.), the DNA fragments were ligated to one another: To a 30µl annealing solution, a 30µl solution II was added and the mixture was thoroughly mixed. A 60µl solution I was then added to the mixture, and the reaction was allowed to proceed at 16°C for 1 hour to ligate the DNA fragments. After treatment with phenol, the aqueous layer was collected and twice as much ethanol was added. The resulting mixture was cooled to -70°C and was then centrifuged to precipitate DNA. The resultant DNA fragments were then subjected to phosphorylation by T4 polynucleotide kinase (Takara Shuzo, Co., Ltd.) to obtain the desired structural gene.

To construct an expression vector, pTCII (See, WO 00/20643) was digested with NdeI and BamHI (Takara Shuzo, Co., Ltd.) at 37°C for 4 hours and the digests were subjected to 1% agarose gel electrophoresis. Using QIAquick gel extraction kit (QIAGEN), 4.4kb DNA fragments were then extracted and were dissolved in a 25µl TE buffer solution. Using TaKaRa DNA ligation kit ver. 2 (Takara Shuzo, Co., Ltd.), the NdeI and BamHI fragments of pTCII were ligated to the structural gene obtained above. Using 10µl of this reaction mixture, E. coli JM109 competent cells (TOYOBO) were transfected and were plated onto an LB agar medium containing 10µg/ml tetracycline. The culture was incubated at 37°C overnight and tetracycline-resistant colonies were selected. The transformants were then cultured in LB agar medium overnight and, using QIAprep 8 miniprep kit (QIAGEN), the plasmid pTCGPR1 was obtained. The pTCGPR1 so obtained was digested with MunI and BamHI (Takara Shuzo, Co., Ltd.) at 37°C for 4 hours. Subsequently, the digests were subjected to 1% agarose gel electrophoresis and, using QIAquick gel extraction kit (QIAGEN), 4.5kb DNA fragments were extracted and were dissolved in a 25µl TE buffer solution.

Each of DNA oligomers #6, #7, #8, and #9 was allowed to react in a 25µl phosphorylation reaction solution (10µg DNA oligomer, 50mM Tris-HCl, pH7.6, 10mM MgCl₂, 1mM spermidine, 10mM DTT, 0.1mg/ml BSA, 1mM ATP, 10 units of T4 polynucleotide kinase (Takara Shuzo, Co., Ltd.)) at 37°C for 1 hour to phosphorylate 5'-end of each olygomer. After treatment with phenol, twice as much ethanol was added and the mixture was cooled to -70°C and was then centrifuged to precipitate DNA. The resulting DNA fragments were mixed with DNA oligomers #5 and #10 to make a 120µl mixture. The mixture was then maintained at 90°C for 10 minutes and was allowed to slowly cool to room temperature for annealing. Using TaKaRa DNA ligation kit ver. 2 (Takara Shuzo, Co., Ltd.), the DNA fragments were ligated to one another: To a 30µl annealing solution, a 30µl solution II was added and the mixture was thoroughly mixed. A 60µl solution I was then added to the mixture, and the reaction was allowed to proceed at 37°C for 1 hour to ligate the DNA fragments. After treatment with phenol, the aqueous layer was collected and twice as much ethanol was added. The resulting mixture was cooled to -70°C and was then centrifuged to precipitate DNA. The resultant DNA fragments were then subjected to phosphorylation by T4 polynucleotide kinase (Takara Shuzo, Co., Ltd.). The structural gene segment so obtained was ligated to the MunI and BamHI fragments of pCTGPR1. Using 10µl of this reaction mixture, E. coli JM109 competent cells (TOYOBO) were transfected and were plated onto an LB agar medium containing 10µg/ml tetracycline. The culture was incubated at 37°C overnight and tetracycline-resistant colonies were selected. The transformants were then cultured in LB agar medium overnight and, using QIAprep 8 miniprep kit (QIAGEN), the plasmid pTCGPR3 was obtained. Using a DNA sequencer (model 377, Applied Biosystems Ltd.), the base sequence of the structural gene of the pTCGPR3 encoding the precursor protein was confirmed. E. coli strain MM294 (DE3) was then transfected with the plasmid pTCGPR3 and, as a result, the MM294 (DE3)/pTCGPR3 strain, which can express the desired precursor protein, was obtained.

### (b) Preparation of precursor protein

MM294 (DE3)/pTCGPR3 cells were placed in a 200ml flask along with 30mL LB medium (1% peptone, 0.5% yeast extract, and 05% sodium chloride) containing 10mg/L tetracycline and were shake-cultured at 37°C for 8 hours. 15ml of the resulting culture solution was transferred to a 1000ml flask containing 300ml main fermentation medium (1.68% sodium monohydrogen phosphate, 0.3% potassium dihydrogen phosphate, 0.1% ammonium chloride, 0.05% sodium chloride, 0.025% magnesium sulfate, 0.00025% thiamine hydrochloride, 1.5% glucose, and 1.5% casamino acid) and was shake-cultured at 37°C. Once the turbidity of the culture solution reached 150 Klett units, isopropyl-β-D-thiogalactopyranoside was added to a final concentration of 10mg/L. The cells were then cultured for additional 3 hours. Subsequently, the culture solution (300ml) was centrifuged to obtain about 2g of wet cells.

### Example 2

2g of the cells obtained in Example 1 were sonicated (Model 450, BRANSON SONIFIER) in 10ml 10mM EDTA (pH6.0) and were then centrifuged (15000rpm, 15min). The same process was repeated for the resulting precipitate. The precipitate was then stirred for 2 hours in a 5ml 7M guanidine solution (pH5.0) and the mixture was centrifuged (15000rpm, 15min). To the supernatant, 17mg Tris(2-carboxyethyl)-phosphine hydrochloride (TCEP-HC1) was added and the solution was reduced at 50°C for 10 minutes. Subsequently, the solution was loaded onto a C4P-50 column (1cm x 25cm, SHOWA DENKO K. K.) equilibrated with 0.1% TFA to allow the proteins to be adsorbed. After washing, the bound proteins were eluted with a 20-60% step gradient of buffer B (buffer B: 80% acetonitrile/0.1% trifluoroacetic acid) at a flow rate of 2ml/min. The fraction containing the precursor protein (eluted approximately after 27 minutes) was collected and was freeze-dried to obtain a freeze-dried powder of the precursor protein.

### Example 3

The freeze-dried powder of the precursor protein obtained in Example 2 was dissolved in a 1ml solution of 6M urea/0.1M acetic acid. To the resulting mixture, 3.5mg DMAP-CN was added and the reaction was allowed to proceed at 25°C for 15 minutes. Subsequently, the solution was loaded onto a C4P-50 column (1cm x 25cm, SHOWA DENKO K. K.) equilibrated with 0.1% TFA to allow the proteins to be adsorbed. After washing, the bound proteins were eluted with a 20-60% step gradient of buffer B (buffer B: 80% acetonitrile/0.1% trifluoroacetic acid) at a flow rate of 2ml/min. The fraction containing the S-cyanylated precursor protein (eluted approximately after 27 minutes) was collected and was freeze-dried to obtain a freeze-dried powder of the S-cyanylated precursor protein. The freeze-dried powder was dissolved in a 0.8ml 6M solution of urea. A 0.2ml 1N solution of caustic soda was then added to the solution and the reaction was allowed to proceed at 0°C for 15 minutes. Subsequently, acetic acid was added to adjust the pH of the reaction mixture to 7.4. To the resulting mixture, a 9ml solution containing 50mM NaCl, 2mM CaCl₂ and 20mM Tris-HCl (pH7.4), and then, 10 units enterokinase (Navagen) were added and the reaction was allowed to proceed at 25°C for 20 hours. Subsequently, the solution was loaded onto a C4P-50 column (1cm x 25cm, SHOWA DENKO K. K.) equilibrated with 0.1% trifluoroacetic acid to allow the proteins to be adsorbed. After washing, the bound proteins were eluted with a 20-60% step gradient of buffer B (buffer B: 80% acetonitrile/0.1% trifluoroacetic acid) at a flow rate of 2ml/min. The fraction containing hGPR8L (eluted approximately after 22 minutes) was collected and was freeze-dried to obtain approximately 70µg of a freeze-dried powder of hGPR8L.

### Example 4 (Characterization of hGPR8L)

### a) Analysis of N-terminal amino acid sequence

Amino acid sequence of N-terminal region of the product obtained above was determined by a gas phase protein sequencer (model 491, PE Applied Biosystems Ltd.). The determined sequence matched the N-terminal amino acid sequence of hGPR8L expected from its DNA base sequence (Table 1).

**[Table 1]**

| N-terminal amino acid sequence | | |
|---|---|---|
| Residue No. | Detected PTH-amino acids¹⁾ | Amino acids expected from base sequence of hGPR8 ligand |
| 1 | Trp | Trp |
| 2 | Tyr | Tyr |
| 3 | Lys | Lys |
| 4 | His | His |
| 5 | Val | Val |
| 6 | Ala | Ala |
| 7 | Ser | Ser |
| 8 | Pro | Pro |
| 9 | Arg | Arg |
| 10 | Tyr | Tyr |

| | | |
|---|---|---|
| 1) phenylthiohydantoin | | |

### b) Mass spectrometry

Mass spectrometry of the hGPR8L obtained above indicated that the protein had a mass of 2583.7Da (theoretical value: 2584.0).

### C) Activity

Using a fraction containing cell membrane of CHO cells expressing GPR8, the GTPγS binding activity of the hGPR8L obtained above was determined by the same process as described' in Example 6 in WO 01/98494. The results indicated that the protein had a binding activity equivalent to that of the artificially synthesized protein.

### Example 5

### Preparation of plasmid expressing precursor protein of peptide KiSS-1 in E. coli

A plasmid for expressing the precursor protein of peptide Kiss-1 (35-54), which contains amino acids 35 to 54 of the amino acid sequence of SEQ ID NO: 1, can be constructed as follows:

Using the same technique as described in Example 1, a structural gene encoding three tandem repeats of peptide KiSS-1 (35-54) is prepared from 10 different DNA fragments. Using TaKaRa DNA ligation kit ver. 2 (Takara Shuzo, Co., Ltd.), the structural gene is ligated to NdeI and BamHI fragments of pTCII in a reaction mixture. Using 10µl of this reaction mixture, E. coli JM109 competent cells (TOYOBO) are transfected and are plated onto an LB agar medium containing 10µg/ml tetracycline. The culture is incubated at 37°C overnight and tetracycline-resistant colonies are selected. The transformants are then cultured in LB agar medium overnight and, using QIAprep 8 miniprep kit (QIAGEN), the plasmid pTCKiSS3554 is obtained. The base sequence of the polypeptide-encoding DNA is confirmed by a DNA sequencer (model 377, Applied Biosystems Ltd.). By transfecting E. coli strain MM294(DE3) with the plasmid pTCKiSS3554, MM294(DE3)/pTCKiSS3554 strain, which is capable of expressing the desired precursor protein, is obtained.

### Example 6

### Preparation of precursor protein

E. coli strain MM294(DE3)/pTCKiSS3554 obtained in Example 5 is placed in a 2L flask along with 1L LB medium (1% peptone, 0.5% yeast extract, and 0.5% sodium chloride) containing 5.0mg/L tetracycline and is shake-cultured at 37°C for 8 hours. The resulting culture solution is transferred to a 50L fermentation vessel containing 19L main fermentation medium (1.68% sodium monohydrogen phosphate, 0.3% potassium dihydrogen phosphate, 0.1% ammonium chloride, 0.05% sodium chloride, 0.05% magnesium sulfate, 0.02% anti-foaming agent, 0.00025% ferrous sulfate, 0.0005% thiamine hydrochloride, 1.5% glucose, and 1.5% Hy-Case Amino (trade name)) and is stirred at 30°C while aerated. Once the turbidity of the culture solution reaches 500 Klett units, isopropyl-β-D-thiogalactopyranoside is added to a final concentration of 10mg/L. The cells are then cultured for additional 4 hours. Subsequently, the culture solution is centrifuged to obtain wet cells and the cells are stored at -80°C.

### Example 7

### Collection of KiSS-1 (35-44)

100g of the cells obtained in Example 6 is sonicated (Model 450, BRANSON SONIFIER) in 200ml 10mM EDTA (pH6) and is then centrifuged (10000rpm, 60min). The same process is repeated for the resulting precipitate. The precipitate is then stirred for 2 hours in a 50ml solution of 8M urea/0.1M acetic acid and the mixture is centrifuged (10000rpm, 60min). To the supernatant, 200mg 1-cyano-4-dimethylaminopyridinium salt (DMAP-CN) is added and the reaction is allowed to proceed at room temperature for 15 minutes. Subsequently, the solution is loaded onto a Sephadex G-25 column (46mmID x 600mmL, PHARMACIA K. K.) equilibrated with 10% acetic acid and is developed with the same 10% acetic acid used for equilibration at a flow rate of 6ml/min to obtain S-cyanylated polypeptide. Using Pellicon Mini Cassette (Millipore), the eluate is concentrated and desalted, and urea is added to a final concentration of 6M, followed by the addition of 25% aqueous ammonia to a final concentration of 3M. The reaction is allowed to proceed at 15°C for 15 minutes. Subsequently, acetic acid is added to adjust the pH of the reaction mixture to 6.0 and the mixture is loaded onto an ODS-120T column (21.5mm x 300mm, TOSOH corporation) equilibrated with 0.1% trifluoroacetic acid (TFA) to allow the proteins to be adsorbed. After washing, the bound proteins are eluted with a 20-60% step gradient of buffer B (buffer B: 80% acetonitrile/0.1% TFA). The fraction containing the peptide with amidated C-terminal is collected and is freeze-dried to obtain a freeze-dried powder of the peptide. The peptide is then dissolved in 10ml 70% formic acid and 10mg cyanogen bromide is added to the solution. The reaction is allowed to proceed at room temperature for 24 hours. Subsequently, the reaction mixture is loaded onto an ODS-120T column (21.5mm x 300mm, TOSOH corporation) equilibrated with 0.1% trifluoroacetic acid (TFA) to allow the peptides to be adsorbed. After washing, the bound peptides are eluted with a 20-60% step gradient of buffer B (buffer B: 80% acetonitrile/0.1% TFA). The fraction containing KiSS-1 (35-45) is collected and is freeze-dried to obtain a freeze-dried powder of KiSS-1 (35-54).

### Example 8

### Preparation of KiSS-1 (45-54)

Using the same technique as described in Example 1, a plasmid pTCKiSS4554, which is capable of expressing a precursor protein of peptide KiSS-1 (45-54) that corresponds to amino acids 45 to 54 of the amino acid sequence of SEQ ID NO: 1, is constructed. The plasmid contains a structural gene that encodes nine tandem repeats of Kiss-1 (45-54). By transfecting E. coli strain MM294 (DE3) with the plasmid pTCKiSS4554, the MM294 (DE3)/pTCKiSS4554 strain, which is capable of expressing the desired precursor protein, is obtained. In the same manner as in Example 6, this expression strain is cultured and the expression of the peptide is induced by isopropyl-β-D-thiogalactopyranoside to obtain KiSS-1 (45-54).

### INDUSTRIAL APPLICABILITY

According to the method of the present invention, excision of peptides of interest is achieved by the use of the right-handed scissors (*i.e.*, S-cyanylation reaction) along with the left-handed scissors (*i.e.*, treatment with cyanogen bromide, enterokinase, factor Xa, or the like). The method utilizes the excision technique in conjunction with the tandem repeat technique and thereby provides a useful gene recombination-based technique for peptide synthesis that is particularly effective in the large-scale synthesis of low-molecular weight peptides.

## Claims

1. A process for producing a peptide of interest or a salt thereof, **characterized in that**:
a precursor protein, containing repetitive links of the peptides of interest, each of which has an enzymatic or chemical cleavage site added to its N-terminal end and C-terminal end to repetitively link, is enzymatically or chemically cleaved.

2. The process according to claim 1, **characterized in that**:
the precursor protein containing repetitive links of the peptides of interest, each of which has an enzymatic or chemical cleavage site added to its N-terminal end and a chemical cleavage site added to its C-terminal end to repetitively link, is enzymatically or chemically cleaved.

3. A process for producing a peptide of interest or a salt thereof, **characterized in that**:
a precursor protein containing repetitive links of the peptides of interest, each of which has a methionine residue or a protease cleavage sequence added to its N-terminal end and a cysteine residue or a cysteinyl peptide added to its C-terminal end to repetitively link (wherein the peptide moiety of the cysteinyl peptide is a peptide different from the peptide of interest and does not contain any methionine residue when a methionine residue is added to the N-terminal end of each peptide of interest), is cleaved by cyanogen bromide or a protease on the N-terminal side of each peptide and is subjected to a cleavage reaction to cleave on the N-terminal side of each of the C-terminal cysteine residues or the cysteinyl peptides.

4. The process according to claims 1 to 3, wherein the precursor protein is a recombinant precursor protein.

5. The process according to claim 3, wherein the cleavage reaction comprises S-cyanylation reaction, followed by ammonolysis or hydrolysis.

6. The process according to claim 5, wherein the S-cyanylation reaction is carried out in the presence of 2-nitro-5-thiocyanobenzoic acid (NTCB), a 1-cyano-4-dimethylamino pyridium salt (DMAP-CN), or CN⁻ ion.

7. The process according to claim 3, wherein the protease is enterokinase, factor Xa, or thrombin.

8. The process according to claim 3, wherein the following conditions are met:
(1) in cases where cyanogen bromide is used, a methionine residue is linked to the N-terminal end of each peptide of interest and any of the peptides of interest does , not contain a methionine residue;
(2) in cases where the protease is enterokinase, an amino acid sequence Asp-Asp-Asp-Asp-Lys is linked to the N-terminal end of each peptide of interest and any of the peptides of interest does not contain the amino acid sequence Asp-Asp-Asp-Asp-Lys;
in cases where the protease is factor Xa, an amino acid sequence Ile-Glu-Gly-Arg is linked to the N-terminal end of each peptide of interest and any of the peptides of interest does not contain the amino acid sequence Ile-Glu-Gly-Arg; and
(4) in cases where the protease is thrombin, an amino acid sequence Gly-Pro-Arg is linked to the N-terminal end of each peptide of interest and any of the peptides of interest does not contain the amino acid sequence Gly-Pro-Arg.

9. The process according to claims 1 to 3, wherein the peptide of interest is peptide KiSS-1.

10. The process according to claims 1 to 3, wherein the peptide of interest is a ligand for GPR8.

11. A process for producing a GPR8 ligand or a salt thereof, **characterized in that**:
a precursor protein containing three repetitive likings of the GPR8 ligands, each of which has an enterokinase cleavage sequence added to its N-terminal end and a cysteine residue added to its C-terminal end to repetitively link, is cleaved by enterokinase on the N-terminal side of each GPR8 ligand and is subjected to a cleavage reaction to cleave on the N-terminal side of each of the C-terminal cysteine residues.

12. The process according to claim 10 or 11, wherein the GPR8 ligand is a polypeptide that contains an amino acid sequence identical or substantially identical to the amino acid sequence of SEQ ID NO: 44.

13. The process according to claim 10 or 11, wherein the GPR8 ligand is a polypeptide having the amino acid sequence of SEQ ID NO: 44, SEQ ID NO: 45, SEQ ID NO: 46, SEQ ID NO: 47, SEQ ID NO: 48, SEQ ID NO: 49, or SEQ ID NO: 50.

14. The process according to claim 10 or 11, wherein the GPR8 ligand is a polypeptide having the amino acid sequence of SEQ ID NO: 44.

15. A DNA containing a DNA segment encoding a precursor protein containing repetitive links of peptides of interest, each of which has a methionine residue or a protease cleavage sequence added to its N-terminal end and a cysteine residue or a cysteinyl peptide added to its C-terminal end to repetitively link (wherein the peptide moiety of the cysteinyl peptide is a peptide different from the peptide of interest and does not contain any methionine residue when a methionine residue is added to the N-terminal end of each peptide of interest).

16. A recombinant vector containing the DNA segment of claim 15.

17. The recombinant vector according to claim 16, incorporated in an E. coli transformant MM294(DE3)/pTCGPR3 designated as FERM BP-8023.

18. A transformant transformed by the recombinant vector of claim 16.

19. The transformant according to claim 18, which is an E. coli transformant MM294(DE3)/pTCGPR3 designated as FERM BP-8023.

20. A precursor protein or its salt containing repetitive links of peptides of interest, each of which has a methionine residue or a protease cleavage sequence added to its N-terminal end and a cysteine residue or a cysteinyl peptide added to its C-terminal end to repetitively link (wherein the peptide moiety of the cysteinyl peptide is a peptide different from the peptide of interest and does not contain any methionine residue when a methionine residue is added to the N-terminal end of each peptide of interest).

21. The process according to claim 4, wherein the precursor protein is a recombinant precursor protein produced by culturing the transformant of claim 18.
